(19) Europäisches Patentamt
European Patent Office
Office européen des brevets

(11) EP 0 572 894 B1

(12) EUROPÄISCHE PATENTSCHRIFT

(45) Veröffentlichungstag und Bekanntmachung des Hinweises auf die Patenterteilung:
**04.08.1999 Patentblatt 1999/31**

(51) Int Cl.[6]: **G03C 7/305**, G03C 7/34

(21) Anmeldenummer: **93108361.2**

(22) Anmeldetag: **24.05.1993**

# (54) Farbfotografisches Aufzeichnungsmaterial mit einem Cyan-DIR-Kuppler

Color photographic recording material with a cyan-DIR-coupler

Produit photographique d'enregistrement en couleur avec un coupleur DIR cyan

(84) Benannte Vertragsstaaten:
**DE FR GB**

(30) Priorität: **03.06.1992 DE 4218307**
**05.08.1992 DE 4225923**

(43) Veröffentlichungstag der Anmeldung:
**08.12.1993 Patentblatt 1993/49**

(73) Patentinhaber: **Agfa-Gevaert AG**
**51373 Leverkusen (DE)**

(72) Erfinder:
- **Bergthaller, Peter, Dr.**
**W-5060 Bergisch Gladbach 2 (DE)**
- **Bell, Peter, Dr.**
**W-5000 Köln 21 (DE)**

(56) Entgegenhaltungen:
**EP-A- 0 401 612**
**EP-A- 0 442 029**
**EP-A- 0 572 887**
**DE-A- 2 414 006**
**DE-A- 2 454 329**
**DE-A- 3 209 486**
**US-A- 3 617 291**

- **Angewandte Chemie, 103/12 (1991), Seiten 1742-3**

Bemerkungen:
Die Akte enthält technische Angaben, die nach dem Eingang der Anmeldung eingereicht wurden und die nicht in dieser Patentschrift enthalten sind.

EP 0 572 894 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein farbfotografisches Aufzeichnungsmaterial mit mindestens einer lichtempfindlichen Silberhalogenidemulsionsschicht, der ein farbloser Cyan-DIR-Kuppler der nachstehend angegebenen Formel Ia zugeordnet ist.

Es ist bekannt, die chromogene Entwicklung in Gegenwart von Verbindungen durchzuführen, die bei der Entwicklung bildmäßig diffusionsfähige Substanzen freisetzen, die die Entwicklung von Silberhalogenid zu inhibieren vermögen. Derartige Verbindungen werden als sogenannte DIR-Verbindungen (DIR = development inhibitor releasing) bezeichnet. Bei den DIR-Verbindungen kann es sich um solche handeln, die unter Abspaltung eines Inhibitorrestes mit dem Oxidationsprodukt eines Farbentwicklers zu einem Farbstoff reagieren (DIR-Kuppler), oder um solche, die den Inhibitor freisetzen ohne gleichzeitig einen Farbstoff zu bilden. Letztere werden auch als DIR-Verbindungen im engeren Sinne bezeichnet.

Durch Anwendung von DIR-Verbindungen (im weiteren Sinne) kann eine Vielzahl von fotografischen, die Bildqualität beeinflussenden Effekten bewirkt werden. Solche Effekte sind beispielsweise die Erniedrigung der Gradation, die Erzielung eines feineren Farbkorns, die Verbesserung der Schärfe durch den sogenannten Kanteneffekt und die Verbesserung der Farbreinheit und der Farbbrillanz durch sogenannte Interimageeffekte. Zu verweisen ist beispielsweise auf die Publikation "Development-Inhibitor-Releasing (DIR) Couplers in Color Photography" von C.R. Barr, J.R. Thirtle und P.W. Vittum, Photographie Science and Engineering 13, 74 (1969).

Die farblos kuppelnden DIR-Verbindungen haben vor den farbig kuppelnden DIR-Kupplern den Vorteil, daß sie universell einsetzbar sind, so daß die gleiche Verbindung ohne Rücksicht auf die zu erzeugende Farbe in allen lichtempfindlichen Schichten eines farbfotografischen Aufzeichnungsmaterials verwendet werden kann. Dem steht als Nachteil gegenüber, daß farblos kuppelnde DIR-Verbindungen im allgemeinen weniger reaktiv sind. DIR-Kuppler können dagegen wegen der aus ihnen erzeugten Farbe meist nur in einem Teil der lichtempfindlichen Schichten verwendet werden, falls nicht die auf sie zurückzuführende Farbnebendichte in den anderen Schichten tolerierbar oder gar erwünscht ist.

DIR-Kuppler sind beispielsweise bekannt aus US-A 3 148 062, US-A 3 227 554, US-A 3 615 506, US-A 3 617 291 und DE-A 24 14 006.

Bei den freigesetzten Entwicklungsinhibitoren handelt es sich in der Regel um heterocyclische Mercaptoverbindungen oder um Derivate des Benzotriazols. Gelb-DIR-Kuppler, die als Entwicklungsinhibitor monocyclische Triazole freisetzen, sind beispielsweise beschrieben in DE-A 28 42 063 und EP-A 0 272 573.

Die bisher verfügbaren Cyan-DIR-Kuppler lassen noch zu wünschen übrig, weil mit ihnen zwar eine vergleichbare Inhibierung der Schicht, in der der Inhibitor freigesetzt wird, aber nur unzureichende Interimageeffekte (IIE) erzielt werden können. Dies ist möglicherweise darauf zurückzuführen, daß der in einer Silberhalogenidemulsionsschicht freigesetzte Inhibitor nicht in ausreichendem Maß in andere Silberhalogenidemulsionsschichten zu diffundieren vermag. Durch Einbau eines sogenannten timing-Gliedes zwischen Kupplungsstelle des Kupplers und Inhibitorrest ist es möglich zu DIR-Kupplern zu gelangen, deren Inhibitor eine vergleichsweise hohe diffusibility hat. Dies bedeutet aber einerseits höhere Synthesekosten und andererseits wegen des höheren Molekulargewichtes der sogenannten timing-DIR-Kuppler eine höhere Schichtbelastung.

Cyan-DIR-Kuppler, bei denen der Inhibitor über ein Ring-N-Atom unmittelbar an die Kupplungsstelle gebunden ist, sind beispielsweise in DE-A 24 14 006 durch die allgemeine Formel (III) beschrieben, waren aber bisher wegen fehlender Synthesemöglichkeiten nicht verfügbar.

Gegenstand der Erfindung ist ein farbfotografisches Aufzeichnungsmaterial mit mindestens einer auf einen Schichtträger aufgetragenen lichtempfindlichen Silberhalogenidemulsionsschicht, der ein farbloser Cyan-DIR-Kuppler zugeordnet ist, dadurch gekennzeichnet, daß der farblose Cyan-DIR Kuppler der folgenden Formel (Ia) entspricht

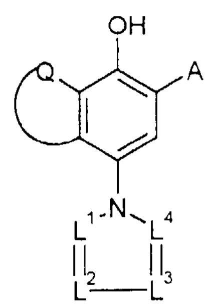

(Ia)

worin bedeuten

A eine Carbamoylgruppe, eine Sulfamoylgruppe oder eine Acylaminogruppe.

Q einen Rest zur Vervollständigung eines ankondensierten Benzolringes;

$L^1$, $L^2$, $L^3$, $L^4$ Ringlieder, von denen zwei für je ein N-Atom und die beiden anderen für je eine Gruppe

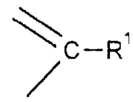

stehen, worin $R^1$ für H, Alkyl, Alkylthio, Aryl, eine heterocyclische Gruppe oder -COOR$^2$ (mit $R^2$ = Alkyl oder Aryl) steht.

Die durch A dargestellten Gruppen können Ballastreste enthalten, z.B. langkettige Alkylreste und/oder Phenyl- oder Phenoxygruppen, die beispielsweise mit Alkyl-, Alkoxy-, Alkoxycarbonyl-, Alkylamino-, Acylamino-, Sulfonamido-, Carbamoyl- und/oder Sulfamoylgruppen und/oder mit Halogenatomen, z.B. Cl substituiert sein können.
Der Durch Q vervollständigte ankondensierte Benzolring kann beispielsweise durch Acylamino-, Sulfonamido-, Alkoxycarbonylamino-, Amino- oder Hydroxylgruppen substituiert sein.
Der durch $L^1$, $L^2$, $L^3$ und $L^4$ vervollständigte Triazolring ist wesentlicher Bestandteil eines Silberhalogenidentwicklungsinhibitors, der über ein N-Atom an die Kupplungsstelle des durch Formel Ia dargestellten Cyankupplers gebunden ist und daraus bei der Farbentwicklung bildmäßig freigesetzt wird. In einer bevorzugten Ausführungsform der vorliegenden Erfindung weist der Inhibitor mindestens eine der folgenden Besonderheiten auf:

a) Er enthält eine im alkalischen Entwickler verseifbare Gruppe.

b) Er hat eine diffusibility $\geq$ 0,4.

Entwicklungsinhibitoren mit im alkalischen Entwickler verseifbaren Gruppen sind beispielsweise beschrieben in DE-A-32 09 486 und DE-A-39 18 394. Bezüglich der Definition der diffusibility und einer Methode zu ihrer Bestimmung ist zu verweisen auf EP-A-0 442 029.
Beispiele für erfindungsgemäße farblose Cyan-DIR-Kuppler sind im folgenden angegeben.

DIR-1

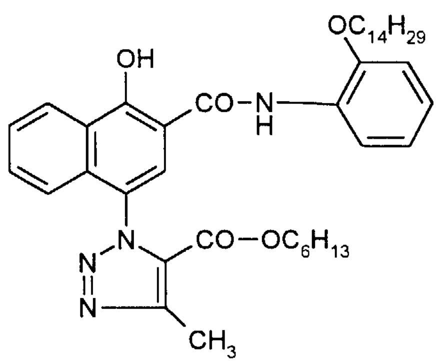

DIR-2

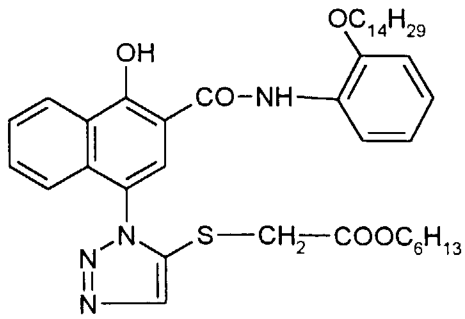

DIR-3

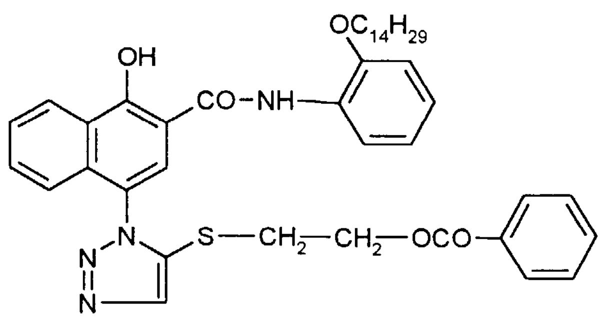

DIR-6

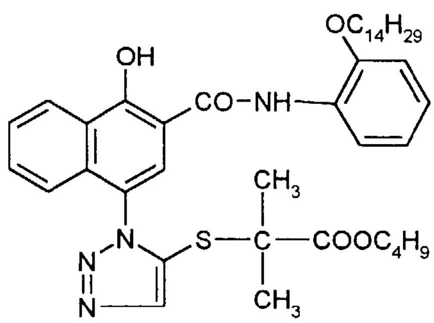

DIR-8

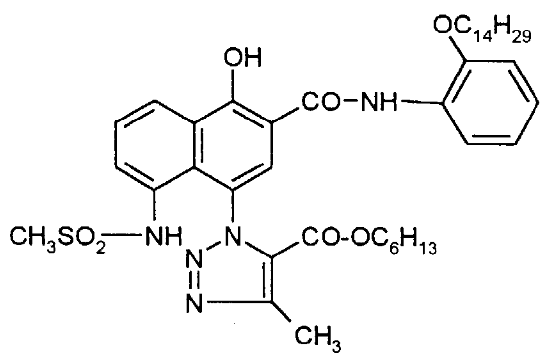

DIR-9

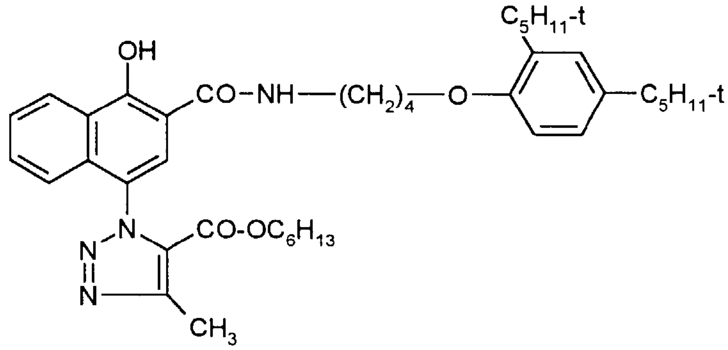

DIR-11

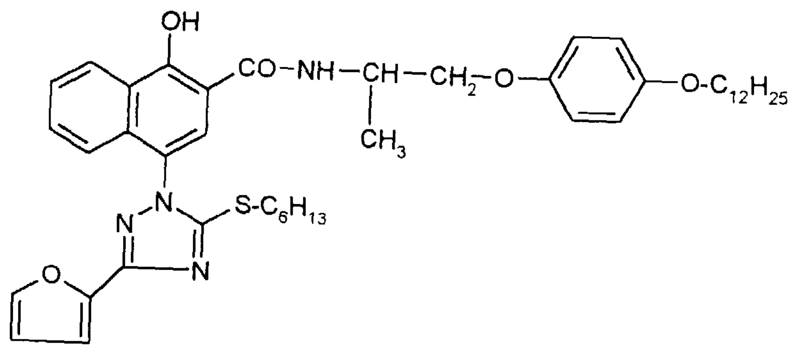

DIR-12

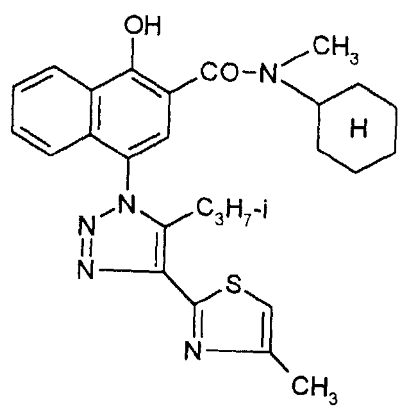

DIR-14

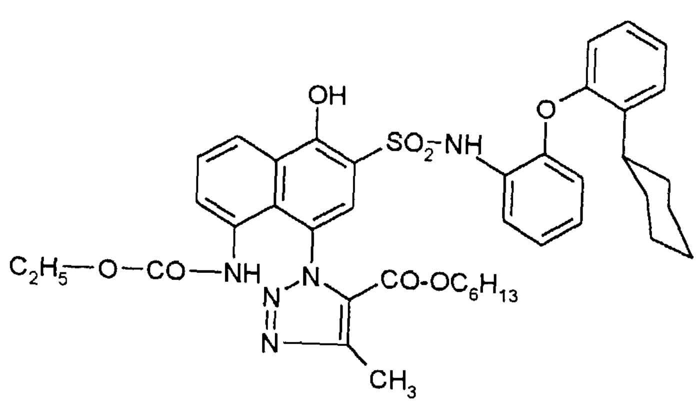

In den obigen Formeln sind die Cyan-DIR-Kuppler dargestellt mit einer Bindung zwischen der Kupplungsstelle des Kupplers und einem der drei N-Atome des Triazolringes. Mit der gewählten Darstellungsweise wird kein Anspruch darauf erhoben, daß die Formeln die tatsächlichen Verhältnisse richtig wiedergeben. Der Triazolring kann auch nach Maßgabe der Herstellungsmethode über eins der beiden anderen N-Atome an die Kupplungsstelle gebunden sein, oder es kann sich um Isomerengemische handeln.

Cyan-DIR-Kuppler der Formel Ia können hergestellt werden nach einem Verfahren, das in der europäischen Patentanmeldung EP-A-0 572 887 beschrieben ist, auf deren Offenbarung hiermit Bezug genommen wird. Das Herstellungsverfahren wird durch folgendes Synthesebeispiel erläutert.

<u>Verbindung DIR-1</u>

1.1

Man setzt 4,75 g (10 mmol) 1-Naphthol-2-carbonsäure-(2-tetradecyloxy)anilid in 70 ml Pyridin mit 2,6 g Iod unter Rühren über 5 h bei Raumtemperatur um. Der iodierter Kuppler kristallisiert beim Einrühren der Lösung in 200 ml 10 %ige Salzsäure aus. Ausbeute 4,4 g. Schmelzpunkt: 72 bis 74°C.

1.2 (Verbindung DIR-1)

Man löst 3 g (5 mmol) iodierten Kuppler aus 1.1 in 200 ml Dichlormethan und gibt unter Rühren 1,55 g (5 mmol) Iodbenzol-bis-trifluoracetat zu. Das Gemisch färbt sich dunkel. Nach Stehen über 1 h und Zugabe von 1,2 g 5-Methyl-1,2,3-triazol-4-carbonsäure-n-hexylester und 1,2 g Tetramethylguanidin wird die Lösung 10 h unter Feuchtigkeitsausschluß stehenlassen. Man gibt 20 ml 10 %iger Salzsäure zu, trennt die Phasen und wäscht die Dichlormethanphase mit Wasser. Nach Trocknen über Natriumsulfat wird eingedampft, in Toluol aufgenommen und über 100 g Kieselgel mit Cyclohexan-Toluol-Ethylacetat-Gemischen bei steigendem Anteil Toluol und Ethylacetat chromatographiert.

Nach Abtrennung zweier vorlaufender Flecke werden aus den polaren Eluaten 1,2 g Verbindung DIR-1 mit dem Schmelzpunkt 82 bis 84°C (aus Cyclohexan) erhalten.
$^1$H-NMR (200 Mhz, $CDCl_3$, TMS)

$\delta$ = 14,03 (s, OH), 8,82 (s, NH), 8,18-8,22 (2d, CH), 8,04-8,08 (d, CH), 7,82 (s, CH), 7,6-7,77 (m, CH), 6,9-7,36 (m, CH), 4,38-4,5 (t, $CH_2$), 4,02-4,15 (t, $CH_2$), 2,7-2,75 (s, $CH_3$), 1,9-1,97 (m, $CH_2$), 1,15-1,5 (m, $CH_2$, $CH_3$), 0,8-0,95 (m, $CH_3$)

In dem erfindungsgemäßen farbfotografischen Aufzeichnungsmaterial, das in der Regel mindestens eine rotempfindliche Silberhalogenidemulsionsschicht mit einem dieser zugeordneten Cyankuppler, mindestens eine grünempfindliche Silberhalogenidemulsionsschicht mit einem dieser zugeordneten Magentakuppler und mindestens eine blauempfindliche Silberhalogenidemulsionsschicht mit einem dieser zugeordneten Gelbkuppler enthält, ist der erfindungsgemäße farblose Cyan-DIR-Kuppler der Formel (Ia) einer oder mehreren dieser lichtempfindlichen Silberhalogenidemulsionsschichten und bevorzugt mindestens einer rotempfindlichen Silberhalogenidschicht zugeordnet. In besonderen Ausgestaltungen der Erfindungen kann ein farbloser Cyan-DIR-Kuppler der Formel (Ia) auch mindestens einer der grünempfindlichen und/oder mindestens einer der blauempfindlichen Schichten zugeordnet sein. Farbfotografische Aufzeichnungsmaterialien, bei denen ein farbloser Cyan-DIR-Kuppler in einer grünempfindlichen und/oder blauempfindlichen Silberhalogenidemulsionsschicht enthalten ist, sind beispielsweise beschrieben in DE-A 24 29 250, DE-A 24 54 301 und DE-A 24 54 329.

Für den Aufbau des blaugrünen Teilfarbenbildes enthält das erfindungsgemäße farbfotografische Aufzeichnungsmaterial mindestens einen farblosen Cyankuppler vom Phenol- oder $\alpha$-Naphtholtyp; geeignete Beispiele hierfür sind (allgemeine Formeln (II), (IIIa), (IV) und (V)):

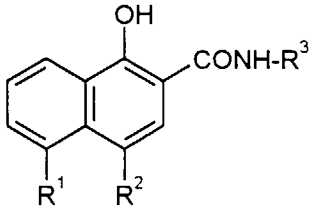

(II)

C-1: $R^1$, $R^2$ = H;

$R^3$ = 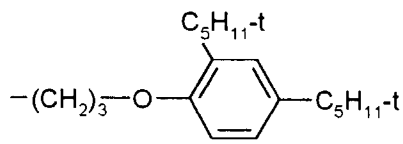

C-2: $R^1$ = $-NHCOOC_4H_9$; $R^2$ = H; $R^3$ = $-(CH_2)_3-OC_{12}H_{25}$

C-3: $R^1$ = H; $R^2$ = $-OCH_2-CH_2-SO_2CH_3$; $R^3$ = $C_{16}H_{33}$

C-4: $R^1$ = H; $R^2$ = $-OCH_2-CONH-(CH_2)_2-OCH_3$;

$R^3$ = 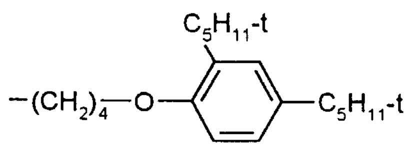

C-5: $R^1$ = $-NH-PO(OC_2H_5)_2$; $R^2$ = H;

$R^3$ = 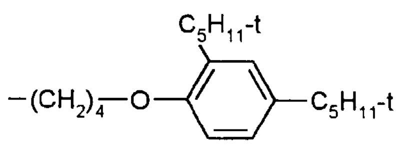

C-6: $R^1$, $R^2$ = H;

$R^3$ = 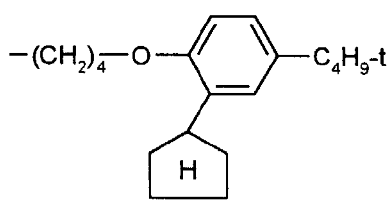

C-7: $R^1$ = H; $R^2$ = Cl; $R^3$ = $-C(C_2H_5)_2-C_{21}H_{43}$

C-8: $R^1$ = H; $R^2$ = $-O-CH_2-CH_2-S-CH(COOH)-C_{12}H_{25}$;
$R^3$ = Cyclohexyl

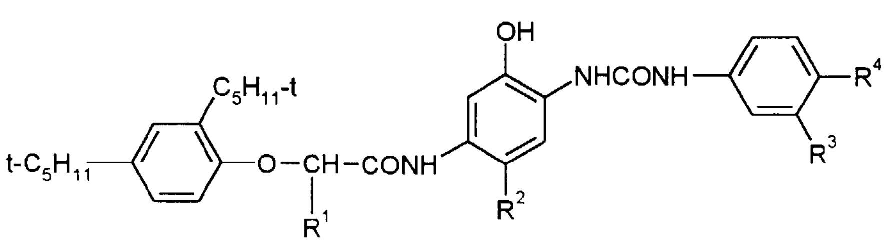

(IIIa)

C-9: $R^1 = -C_4H_9$; $R^2 = H$; $R^3 = H$; $R^4 = -CF_3$

C-10: $R^1 = -C_4H_9$; $R^2 = H$; $R^3 = H$; $R^4 = -SO_2CHF_2$

C-11: $R^1 = -C_4H_9$; $R^2 = -O-CH_2-CONH-(CH_2)_2-OCH_3$; $R^3 = H$; $R^4 = -SO_2-CH_3$

C-12: $R^1 = C_2H_5$; $R^2$, $R^3 = H$; $R^4 = -SO_2CH_3$

C-13 : $R^1 = -C_4H_9$; $R^2$, $R^3 = H$; $R^4 = -SO_2C_4H_9$

C-14: $R^1 = -C_4H_9$; $R^2 = H$; $R^3 = -CN$; $R^4 = -CN$

C-15: $R^1 = -C_4H_9$; $R^2$, $R^3 = H$; $R^4 = -SO_2-CH_2-CHF_2$

C-16: $R^1 = -C_2H_5$; $R^2$, $R^3 = H$; $R^4 = -SO_2CH_2-CHF-C_3H_7$

C-17: $R^1 = -C_4H_9$; $R^2$, $R^3 = H$; $R^4 = F$

C-18: $R^1 = -C_4H_9$; $R^2$, $R^3 = H$; $R^4 = -SO_2CH_3$

C-19: $R^1 = -C_4H_9$; $R^2$, $R^3 = H$; $R^4 = -CN$

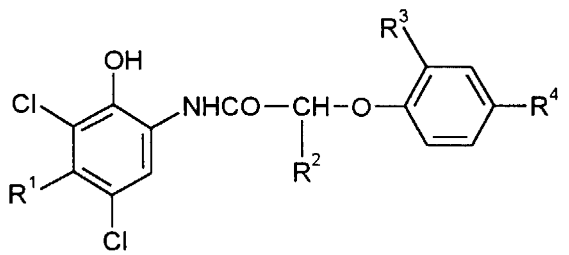

(IV)

C-20: $R^1 = -CH_3$; $R^2 = -C_2H_5$; $R^3$, $R^4 = -C_5H_{11}-t$

C-21: $R^1 = -CH_3$; $R^2 = H$; $R^3$, $R^4 = -C_5H_{11}-t$

C-22: $R^1$, $R^2 = -C_2H_5$; $R^3$, $R^4 = -C_5H_{11}-t$

C-23: $R^1 = -C_2H_5$; $R^2 = -C_4H_9$; $R^3$, $R^4 = -C_5H_{11}-t$

C-24: $R^1 = -C_2H_5$; $R^2 = -C_4H_9$; $R^3$, $R^4 = -C_4H_9-t$

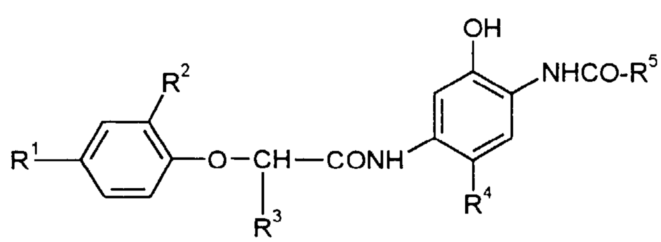

(V)

C-25: $R^1$, $R^2 = -C_5H_{11}-t$; $R^3 = -C_4H_9$; $R^4 = H$; $R^5 = -C_3F_7$

C-26: $R^1 = -NHSO_2-C_4H_9$; $R^2 = H$; $R^3 = -C_{12}H_{25}$; $R^4 = Cl$; $R^5$ = Phenyl

C-27: $R^1$, $R^2$ = $-C_5H_{11}$-t; $R^2$ = Cl, $R^3$ = $-C_3H_7$-i; $R^4$ = Cl;
$R^5$ = Pentafluorphenyl

C-28: $R^1$ = $-C_5H_{11}$-t; $R^2$ = Cl; $R^3$ = $-C_6H_{13}$; $R^4$ = Cl;
$R^5$ = -2-Chlorphenyl

Bevorzugt verwendet werden Cyankuppler der allgemeinen Formeln (IIa) und (III)

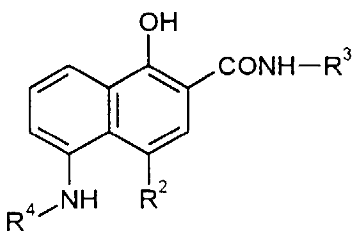

(IIa)

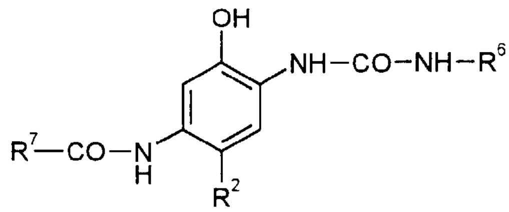

(III)

worin bedeuten

$R^2$ H oder eine unter den Bedingungen der Farbentwicklung freisetzbare fotografisch inaktive Gruppe, die dem Kuppler keine Farbe verleiht;

$R^3$ Alkyl oder Aryl;

$R^4$ H, Acyl, wobei der Acylrest sich von aliphatischen oder aromatischen Carbon- oder Sulfonsäuren, von N-substituierten Carbamin- oder Sulfaminsäuren oder von Kohlensäurehalbestern ableitet, oder

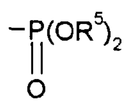 oder 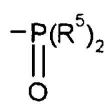 ;

$R^5$ Alkyl;

$R^6$ eine heterocyclische Gruppe oder Aryl;

$R^7$ einen Ballastrest.

Weitere Cyankuppler der Formel (IIa) sind beispielsweise in EP-A 0 161 626 beschrieben. Weitere Cyankuppler der Formel (III) sind beispielsweise in EP-A 0 067 689 und DE-A 3933 899 beschrieben. Es ist insbesondere vorteilhaft einen farblosen Cyan-DIR-Kuppler der Formel (Ia) in Kombination mit einem farblosen Cyankuppler einer der Formeln (II), (III), (IV) und (V) zu verwenden, in denen $R^2$ eine Alkoxygruppe bedeutet. Solche Cyankuppler sind Gegenstand von DE-A 27 47 435.

Das erfindungsgemäße Aufzeichnungsmaterial hat hohe Interimageefekte und ausgezeichnete Farbwiedergabeeigenschaften. Es ist auch hervorragend geeignet für die Verarbeitung in Schnellverarbeitungsprozessen. Insbesondere weist es hierbei eine ausgezeichnete Bleichbarkeit auf Beispielsweise können solche Materialien, die zu einem Quellfaktor von ≤ 3,5, gemessen in Wasser von 10° dH bei 20°C, gehärtet worden sind, in weniger als 3 Minuten vollständig gebleicht werden.

Bei der Herstellung des lichtempfindlichen farbfotografischen Aufzeichnungsmaterials gemäß vorliegender Erfindung werden die erfindungsgemäß verwendeten Cyan-DIR-Kuppler der Formel (Ia) gegebenenfalls zusammen mit anderen Farbkupplern, z.B. den diffusionsfesten farblosen Cyankupplern in bekannter Weise in die Gießlösung der Silberhalogenidemulsionsschichten oder anderer Kolloidschichten eingearbeitet. Beispielsweise können die öllöslichen oder hydrophoben Kuppler vorzugsweise aus einer Lösung in einem geeigneten Kupplerlösungsmittel (Ölbildner) gegebenenfalls in Anwesenheit eines Netz- oder Dispergiermittels zu einer hydrophilen Kolloidlösung zugefügt

werden. Die hydrophile Gießlösung kann selbstverständlich neben dem Bindemittel andere übliche Zusätze enthalten. Die Lösung der Kuppler braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird. Die Zugabe der Kuppler kann auch getrennt erfolgen und die Kuppler müssen auch nicht notwendigerweise der gleichen Schicht zugefügt werden.

Die verwendeten lichtempfindlichen Silberhalogenidemulsionen können als Halogenid Chlorid, Bromid und Iodid bzw. Mischungen davon enthalten. In einer bevorzugten Ausführungsform besteht der Halogenidanteil wenigstens einer Schicht zu 0 bis 20 mol-% aus Iodid, zu 0 bis 50 mol-% aus Chlorid und zu 50 bis 100 mol-% aus Bromid. In einer bevorzugten Ausführungsform handelt es sich um überwiegend kompakte Kristalle, die z.B. kubisch oder oktaedrisch sind oder Übergangsformen aufweisen und im allgemeinen eine durchschnittliche Korngröße von mehr als 0,2 µm aufweisen. Das durchschnittliche Verhältnis von Durchmesser zu Dicke ist bevorzugt kleiner als 8:1, wobei gilt, daß der Durchmesser eines Kornes definiert ist als der Durchmesser eines Kreises mit einem Kreisinhalt entsprechend der projizierten Fläche des Kornes. In einer anderen bevorzugten Ausführungsform können alle oder einzelne Emulsionen aber auch im wesentlichen tafelförmige Silberhalogenidkristalle aufweisen, bei denen das Verhältnis von Durchmesser zu Dicke größer als 8:1 ist. Bei den Emulsionen kann es sich um monodisperse Emulsionen handeln, welche bevorzugt eine mittlere Korngröße von 0,3 µm bis 1,2 µm aufweisen. Die Silberhalogenidkörner können einen geschichteten Kornaufbau aufweisen.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Diese kann jedoch ganz oder teilweise durch andere natürliche oder synthetische Bindemittel ersetzt werden. Begußhilfsmittel und Weichmacher können verwendet werden. Verwiesen wird auf Research Disclosure 17 643 (Dezember 1978), insbesondere Kapitel IX, XI und XII.

Die Emulsionen können in der üblichen Weise chemisch und/oder spektral sensibilisiert sein, sie können weiter mit den üblichen Silberhalogenidstabilisierungsmitteln stabilisiert sein und die Emulsionsschichten wie auch andere nicht-lichtempfindliche Schichten können in der üblichen Weise mit bekannten Härtungsmitteln gehärtet sein. Geeignete chemische Sensibilisatoren, spektrale Sensibilisierungsfarbstoffe, Stabilisatoren und Härtungsmittel sind beispielsweise in Research Disclosure 17643 beschrieben; verwiesen wird insbesondere auf die Kapitel III, IV, VI und X.

Üblicherweise enthalten farbfotografische Aufzeichnungsmaterialien mindestens je eine Silberhalogenidemulsionsschicht für die Aufzeichnung von Licht jedes der drei Spektralbereiche Rot, Grün und Blau. Zu diesem Zweck sind die lichtempfindlichen Schichten in bekannter Weise durch geeignete Sensibilisierungsfarbstoffe spektral sensibilisiert.

Die fotografischen Emulsionen können unter Verwendung von Methinfarbstoffen oder anderen Farbstoffen spektral sensibilisiert werden. Besonders geeignete Farbstoffe sind Cyaninfarbstoffe, Merocyaninfarbstoffe und komplexe Merocyaninfarbstoffe.

Eine Übersicht über die als Spektralsensibilisatoren geeigneten Polymethinfarbstoffe, deren geeignete Kombinationen und supersensibilisierend wirkenden Kombinationen enthält Research Disclosure 17643 (Dez. 1978), Kapitel IV.

Insbesondere sind die folgenden Farbstoffe - geordnet nach Spektralgebieten - geeignet:

1. als Rotsensibilisatoren

   9-Ethylcarbocyanine mit Benzthiazol, Benzselenazol oder Naphthothiazol als basische Endgruppen, die in 5- und/oder 6-Stellung durch Halogen, Methyl, Methoxy, Carbalkoxy, Aryl substituiert sein können sowie 9-Ethylnaphthoxathia- bzw. -selencarbocyanine und 9-Ethyl-naphthothiaoxa- bzw. -benzimidazocarbocyanine, vorausgesetzt, daß die Farbstoffe mindestens eine Sulfoalkylgruppe am heterocyclischen Stickstofftragen.

2. als Grünsensibilisatoren

   9-Ethylcarbocyanine mit Benzoxazol, Naphthoxazol oder einem Benzoxazol und einem Benzthiazol als basische Endgruppen sowie Benzimidazocarbocyanine, die ebenfalls weiter substituiert sein können und ebenfalls mindestens eine Sulfoalkylgruppe am heterocyclischen Stickstoff enthalten müssen.

3. als Blausensibilisatoren

   symmetrische oder asymmetrische Benzimidazo-, Oxa-, Thia- oder Selenacyanine mit mindestens einer Sulfoalkylgruppe am heterocyclischen Stickstoff und gegebenenfalls weiteren Substituenten am aromatischen Kern, sowie Apomerocyanine mit einer Rhodaningruppe.

Als Beispiele seien, insbesondere für Negativ- und Umkehrfilm, die nachfolgend aufgeführten Rotsensibilisatoren RS, Grünsensibilisatoren GS und Blausensibilisatoren BS genannt, die jeweils einzeln oder in Kombination untereinander eingesetzt werden können, z.B. RS-1 und RS-2, sowie GS-1 und GS-2.

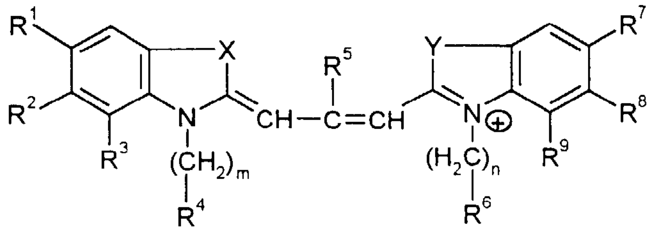

RS-1: $R^1$, $R^3$, $R^7$, $R^9$ = H; $R^2$, $R^8$ = Cl; $R^4$ = $-SO_3^+$-$NH(C_2H_5)_3$;
$R^5$ = $-C_2H_5$; $R^6$ = $-SO_3$-; m, n = 3; X, Y = S;

RS-2: $R^1$, $R^3$, $R^9$ = H; $R^2$ = Phenyl;

$R^4$ = 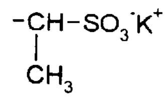 ;

$R^5$ = $-C_2H_5$; $R^6$ = $-SO_3$-; $R^7$, $R^8$ = $-OCH_3$; m = 2; n = 3;
X = 0; Y = S;

RS-3: $R^1$, $R^9$ = H; $R^2$, $R^3$ zusammen -CH=CH-CH=CH-;
$R^4$ = $-SO_3$-$Na^+$; $R^5$ = $-C_2H_5$; $R^6$ = $-SO_3^-$; $R^7$, $R^8$ = Cl;
m, n = 3; X = S; Y = N-$C_2H_5$;

RS-4: $R^1$ = $-OCH_3$; $R^2$, $R^8$ = $-CH_3$; $R^3$, $R^4$, $R^7$, $R^9$ = H;
$R^5$ = $-C_2H_5$; $R^6$ = $-SO_3^-$; m = 2; n = 4; X = S; Y = Se;

RS-5: $R^1$, $R^7$ = H; $R^2$, $R^3$ sowie $R^8$, $R^9$ zusammen -CH=CH-CH=CH-;
$R^4$ = $-SO_3^+$-$NH(C_2H_5)_3$; $R^5$ = $-C_2H_5$; $R^6$ = $SO_3^-$;
m = 2; n = 3; X, Y = S;

GS-1: $R^1$, $R^3$, $R^7$, $R^9$ = H; $R^3$, $R^2$ = Phenyl;

$R^4$ = 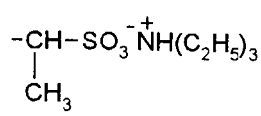 ;

$R^5$ = $-C_2H_5$; $R^6$ = $-SO_3$-;
$R^8$ = Cl; m = 2; n = 3; X, Y = O;

GS-2: $R^1$, $R^2$, $R^7$, $R^8$ = Cl; $R^3$, $R^5$, $R^6$, $R^9$ = H;

$R^4$ = 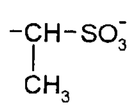 ;

m, n = 2; X, Y = N-$C_2H_5$;

GS-3: $R^1$, $R^7$ = H; $R^2$, $R^3$ sowie $R^8$, $R^9$ zusammen -CH=CH-CH=CH-; $R^4$ = $SO_3^-Na^+$; $R^5$ = $C_2H_5$; $R^6$ = $SO_3^-$; m, n = 3; X, Y = O;

GS-4: $R^1$, $R^3$, $R^4$, $R^7$, $R^8$, $R^9$ = H; $R^2$ = $-OCH_3$; $R^5$ = $-C_2H_5$; $R^6$ = $SO_3^-$; m = 2; n = 4; X = O; Y = S;

BS-1:

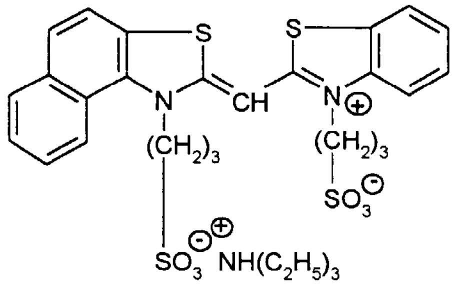

BS-2:

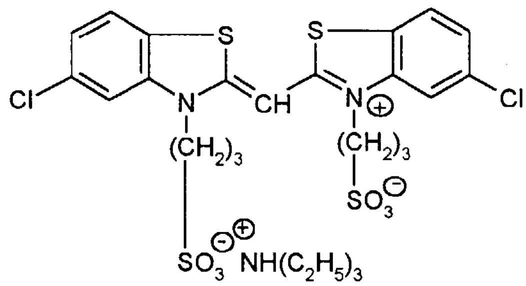

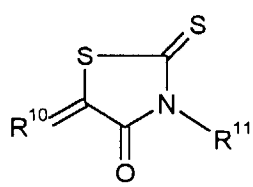

BS-3:

$R^{10}$ = 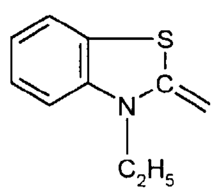 ;

$R^{11}$ = $-CH_2-COOH$;

BS-4:

$R^{10}$ = 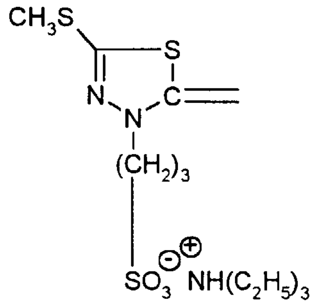 ;

$R^{11} = -C_2H_5$ ;

BS-5:

$R^{10}$ = 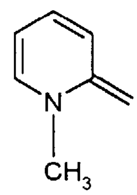 ;

$R^{11} = -C_2H_5$ ;

Auf Sensibilisatoren kann verzichtet werden, wenn für einen bestimmten Spektralbereich die Eigenempfindlichkeit des Silberhalogenids ausreichend ist, beispielsweise die Blauempfindlichkeit von Silberbromiden.

Jede der genannten lichtempfindlichen Schichten kann aus einer einzigen Schicht bestehen oder in bekannter Weise, z.B. bei der sogenannten Doppelschichtanordnung, auch zwei oder auch mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtempfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709; DE-A-25 30 645, DE-A-26 22 922). Solche Silberhalogenidteilschichten gleicher Spektralempfindlichkeit weisen in der Regel unterschiedliche Lichtempfindlichkeit (speed) auf, wobei die empfindlicheren Teilschichten im allgemeinen vom Schichtträger weiter entfernt angeordnet sind als weniger empfindliche Teilschichten gleicher Spektralempfindlichkeit.

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Cyan, Magenta und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nicht lichtempfindlichen Bindemittelschicht.

Unter spektraler Zuordnung ist zu verstehen, daß die Spektralempfindlichkeit jeder der lichtempfindlichen Silberhalogenidemulsionsschichten und die Farbe des aus dem jeweils räumlich zugeordneten Farbkuppler erzeugten Teilfarbenbildes in einer bestimmten Beziehung zueinander stehen, wobei jeder der Hauptspektralempfindlichkeiten (Rot, Grün, Blau) eine andere Farbe des betreffenden Teilfarbenbildes (im allgemeinen z.B. die Farben Cyan, Magenta bzw. Gelb, in dieser Reihenfolge) zugeordnet ist.

Jeder der unterschiedlich spektral sensibilisierten Silberhalogenidemulsionsschichten kann ein oder können

auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdiffundierender Farbkuppler zur Erzeugung des blaugrünen Teilfarbenbildes zugeordnet, und zwar im vorliegenden Fall vorzugsweise mindestens ein farbloser Cyankuppler einer der Formeln (IIa) und (III), und mindestens ein Cyan-DIR-Kuppler der Formel (Ia). Darüberhinaus kann auch eine noch vorhandene rote bzw. purpurfarbene Nebendichte des Cyanfarbstoffes maskiert werden, wenn den rotempfindlichen Schichten zusätzlich einer der üblichen roten Maskenkuppler zugeordnet wird. Solche roten Cyankuppler sind bekannt und beispielsweise in DE-A-25 38 323 beschrieben und schließlich kann auch eine noch vorhandene gelbe Nebendichte wirksam maskiert werden, wenn den rotempfindlichen Schichten zusätzlich ein gelber Maskenkuppler, der bei der chromogenen Entwicklung einen Cyanfarbstoffbildet, zugeordnet wird. Solche gelben Cyankuppler sind beispielsweise beschrieben in DE-A-38 15 469, EP-A-0 442 029, EP-A-0423 727 und DE-A40 17 245.

Grünempfindlichen Silberhalogenidemulsionsschichten ist mindestens ein nichtdiffundierender Farbkuppler zur Erzeugung des purpurnen Teilfarbenbildes zugeordnet und blauempfindlichen Silberhalogenidemulsionsschichten schließlich ist mindestens ein nichtdiffundierenden Farbkuppler zur Erzeugung des gelben Teilfarbenbildes zugeordnet.

Farbkuppler zur Erzeugung des purpurnen Teilfarbenbildes sind in der Regel Kuppler vom Typ des 5-Pyrazolons, des Indazolons oder der Pyrazoloazole; geeignete Beispiele hierfür sind

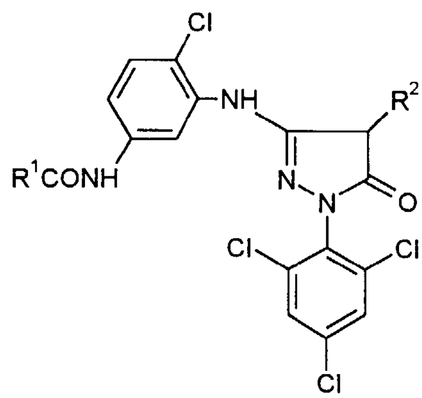

M-1:

$R^1$ = 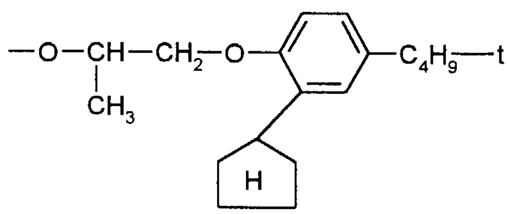 ;

$R^2$ = H

M-2:

$R^1$ = 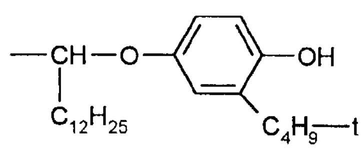 ;

$R^2$ = H

M-3: $R^1$ = $-C_{13}H_{27}$; $R^2$ = H

M-4: $R^1$ = $-OC_{16}H_{33}$; $R^2$ = H

M-5: $R^1$ = -C13$H_{27}$;

$R^2$ = 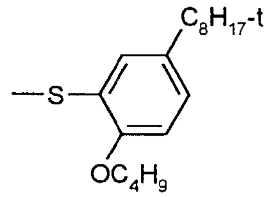

M-6:

$R^1$ = 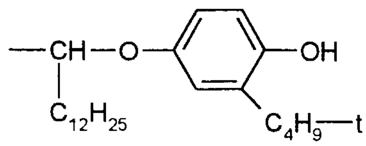 ; $R^2$ = 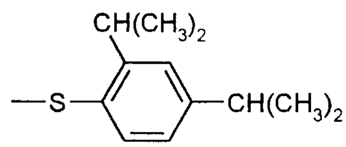 ;

M-7: $R^1$ = $-C_9H_{19}$ ;

$R^2$ = 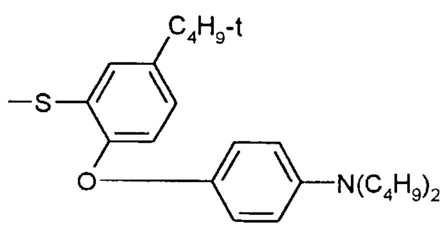

M-8:

$R^1$ = 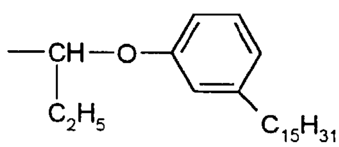 ; $R^2$ = 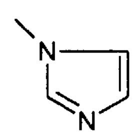 ;

M-9:

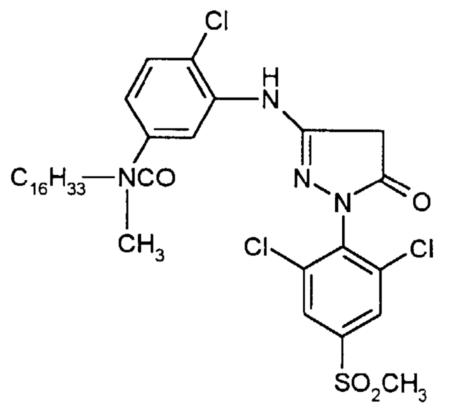

M-10:

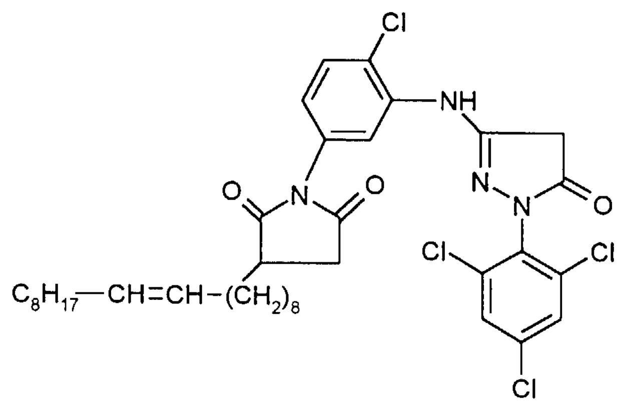

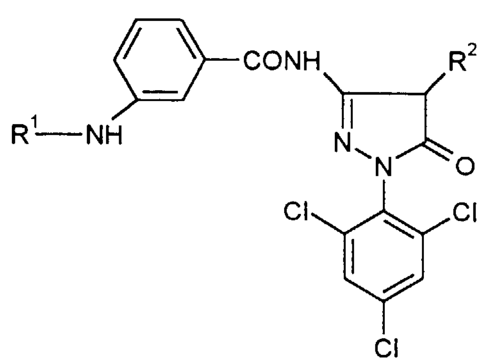

M-11:

$R^1$ = 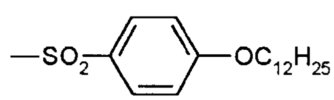 ;

$R^2$ = H

M-12:

$R^1$ = 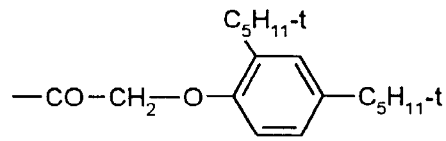 ;

$R^2$ = H

M-13:

$R^1$ = 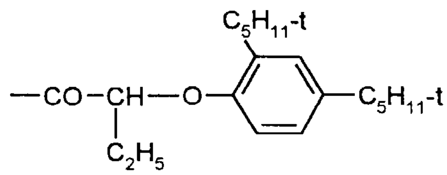 ;

$R^2$ = H

M-14:

$R^1$ = 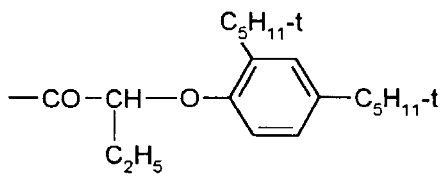 ;

$R^2$ = 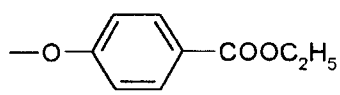

M-15:

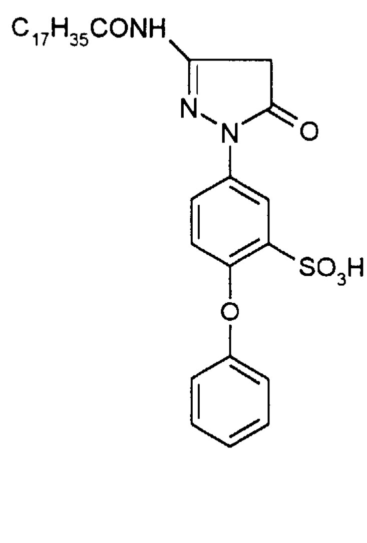

M-16:

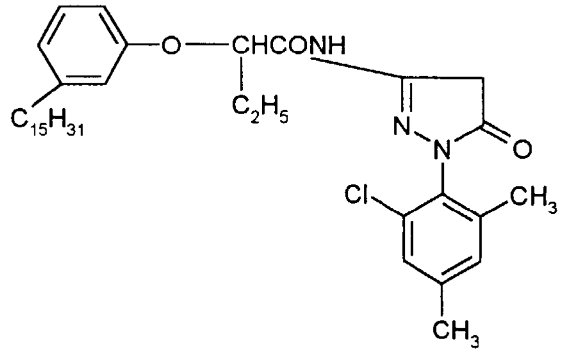

M-17:

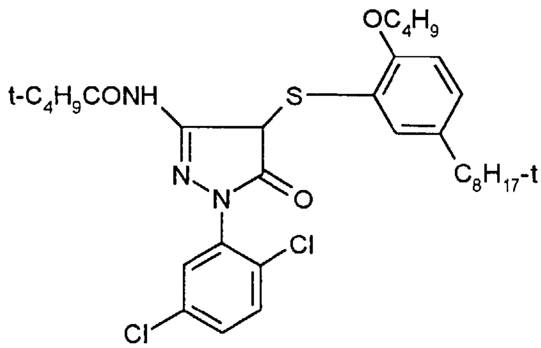

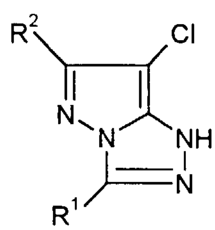

(VIa)

M-18:

$R_1$ = 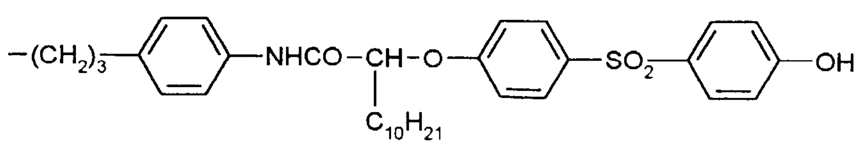

$R^2$ = $-CH_3$

M-19:

$R^1$ = 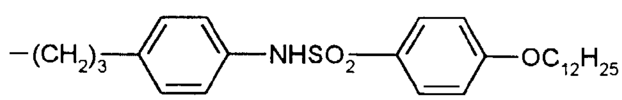

$R^2$ = $-CH_3$

M-20:

$R^1$ = 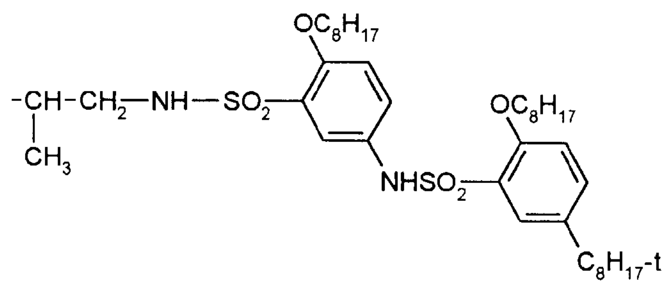

$R^2$ = $-C_4H_9$-t

M-21:

$R^1$ = 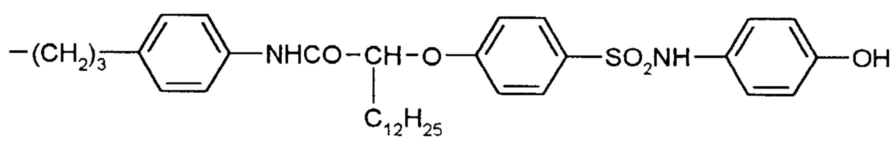

$R^2$ = $-CH_3$

M-22:

$R^1$ = 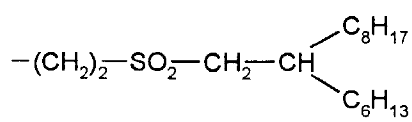

$R^2$ = $-CH_3$

M-23:

$R^1$ = 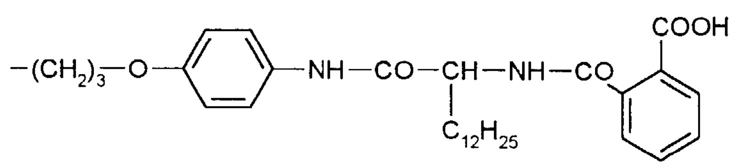

$R^2$ = $-CH_3$

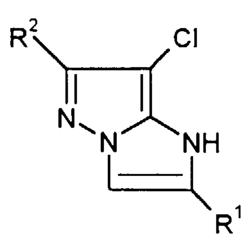

(VIIa)

M-24:

$R^1$ = 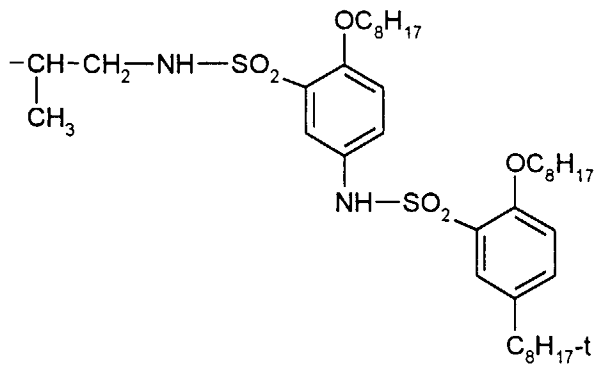

$R^2 = -CH_3$

M-25:

$R^1$ = 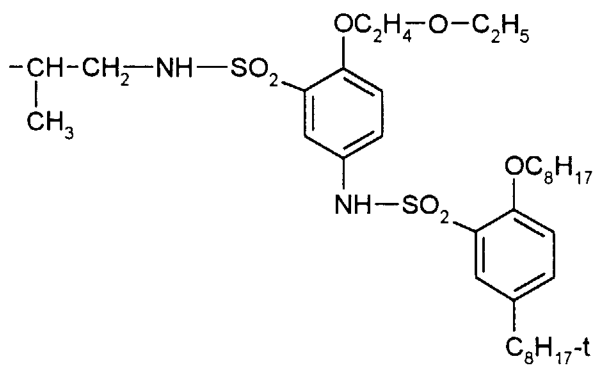

$R^2 = -CH_3$

M-26:

$R^1$ = 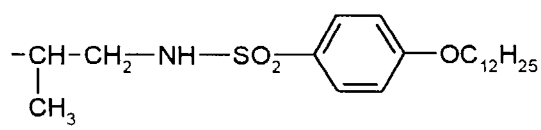

$R^2 = -C_3H_7$-i

M-27:

$R^1$ = 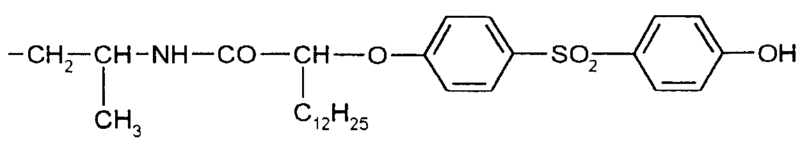

$R^2 = -CH_3$

M-28: $R^1 = -C_3H_7$-i

$R^2$ = 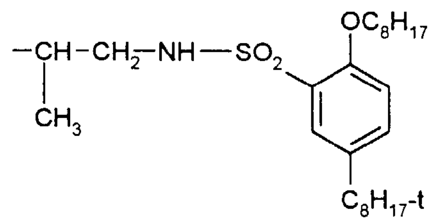

M-29:

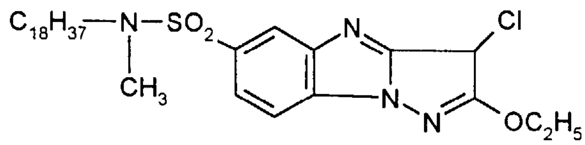

Farbkuppler zur Erzeugung des gelben Teilfarbenbildes sind in der Regel Kuppler mit einer offenkettigen Ketomethylengruppierung, insbesondere Kuppler vom Typ des α-Acylacetamids; geeignete Beispiele hierfür sind a-Benzoylacetanilidkuppler und αPivaloylacetanilidkuppler der Formeln

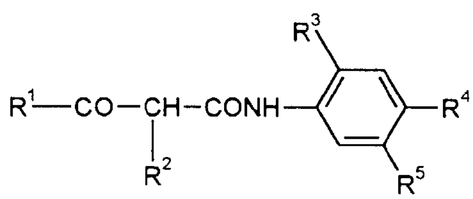

Y-1: $R^1$ = $-C_4H_9$-t;

$R^2$ = 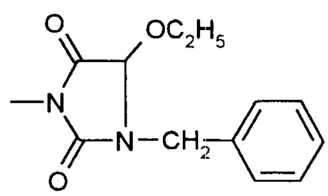 ;

$R^3$ = Cl; $R^4$ = H;

$R^5$ = 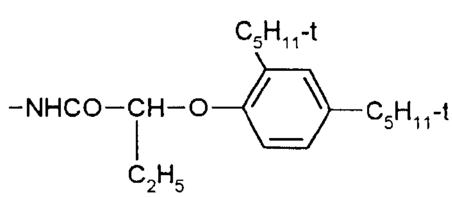

Y-2: $R^1$ = $-C_4H_9$-t;

$R^2 =$ 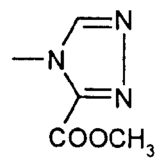 ;

$R^3 = -OC_{16}H_{33}$; $R^4 = H$; $R^5 = -SO_2NHCH_3$

Y-3: $R^1 = -C_4H_9$-t;

$R^2 =$ 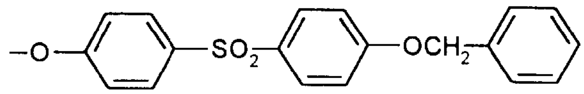 ;

$R^3 = Cl$; $R^4 = H$; $R^5 = -NHSO_2-C_{16}H_{33}$

Y-4:

$R^1 =$ 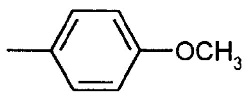

$R^2 =$ 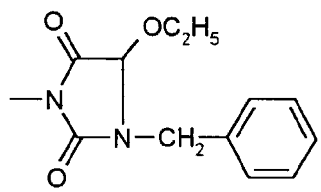 ;

$R^3 = Cl$; $R^4 = H$; $R^5 = -COOC_{12}H_{25}$

Y-5: $R^1 = -C_4H_9$-t;

$R^2 =$ 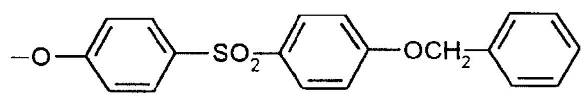 ;

$R^3 = Cl$;

$R^4 = H$; $R^5 =$ 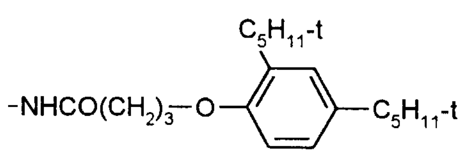

Y-6: $R^1 = -C_4H_9$-t;

$R^2 =$ 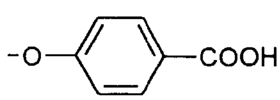 ;

$R^3 = Cl$; $R^4 = H$;

$R^5 =$ 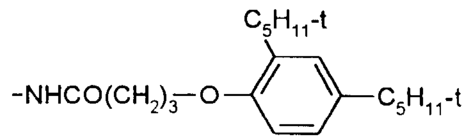

Y-7: $R^1 = -C_4H_9$-t;

$R^2 =$ 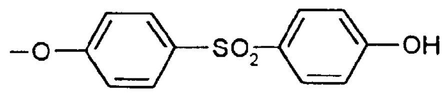 ;

$R^3 = Cl$; $R^4 = H$; $R^5 = -NHSO_2-C_{16}H_{33}$

Y-8: $R^1 = -C_4H_9$-t;

$R^2 =$ 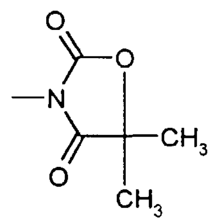 ;

$R^3 = Cl$; $R^4 = H$;

$R^5 =$ 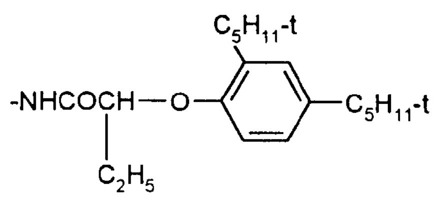

Y-9: $R^1 = -C_4H_9$-t;

$R^2 =$ 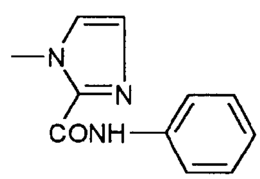 ;

$R^3 = -OC_{16}H_{33}$ ;
$R^4 = H$; $R^5 = -SO_2NHCOC_2H_5$

Y-10: $R^1 = -C_4H_9$-t;

$R^2$ = 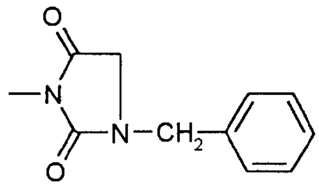 ;

$R^3$ = Cl; $R^4$ = H;

$R^5$ = 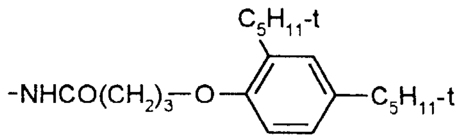

Y-11: $R^1$ = $-C_4H_9$-t;

$R^2$ = 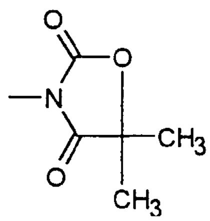 ;

$R^3$ = Cl; $R^4$ = H;

$R^5$ = 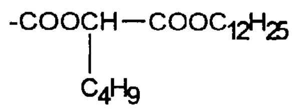

Y-12: $R^1$ = $C_4H_9$-t;

$R^2$ = 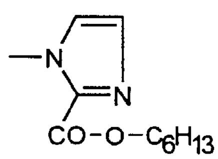 ;

$R^3$ = Cl; $R^4$ = H;

$R^5$ = 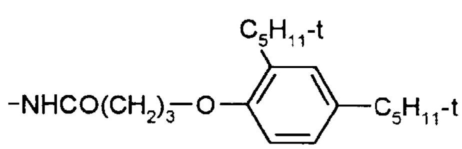

Y-13: $R^1$ = $-C_4H_9$-t;

$R^2 =$ 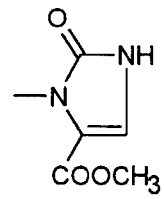 ;

$R^3 = -OC16H_{33}$; $R^4 = H$; $R^5 = -SO_2NHCH_3$

Y-14: $R^1 = -C_4H_9$-t;

$R^2 =$ 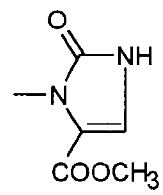 ;

$R^3 = Cl$; $R^4 = H$;

$R^5 =$ 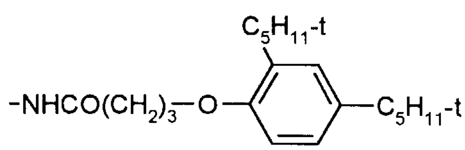

Y-15:

$R^1 =$ 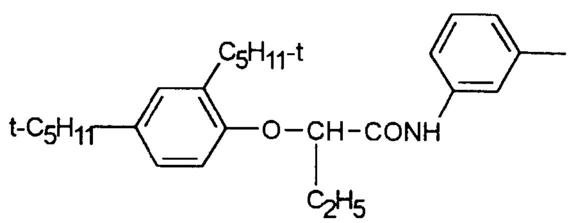 ;

$R^2$, $R^4$, $R^5 = H$; $R^3 = -OCH_3$

Y-16:

$R^1 =$ 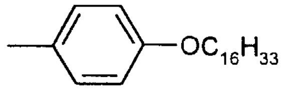 ; $R^2 =$ 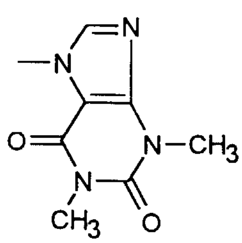 ;

$R^3$, $R^5 = -OCH_3$; $R^4 = H$

Y-17:

$R^1 =$ 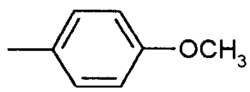 ; $R^2 =$ 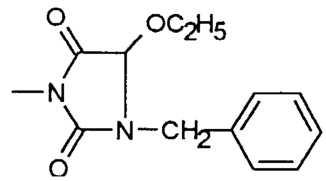 ;

$R^3 = Cl$; $R^4 = H$; $R^5 = -COOC_{12}H_{25}$

Y-18:

$R^1 =$ 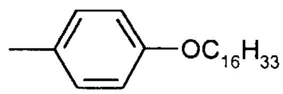 ; $R^2 =$ 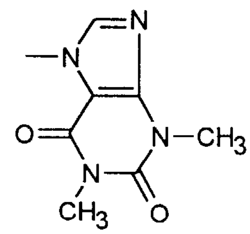 ;

$R^3 = Cl$; $R^4$, $R^5 = -OCH_3$

Y-19:

$R^1 =$ 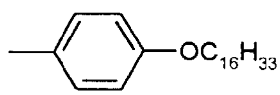 ; $R^2 =$ 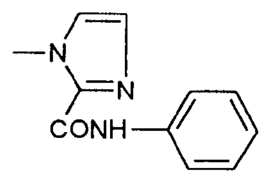 ;

$R^3 = -OCH_3$; $R^4 = H$; $R^5 = -SO_2N(CH_3)_2$

Y-20:

Y-20: $R^1 =$ 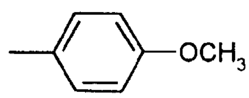 ;

$R^2 =$ 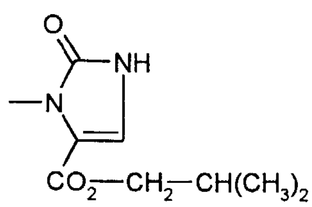 ;

$R^3 = -OCH_3$; $R^4 = H$;

$R^5 =$ 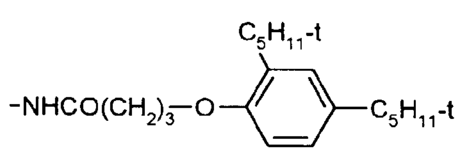

Y-21:

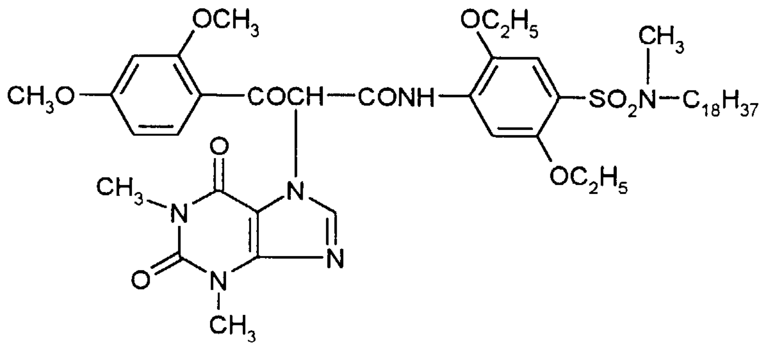

Besonders günstige Ergebnisse hinsichtlich starker und in etwa gleichgroßer Interimageeffekte können erfindungsgemäß erhalten werden, wenn als farbloser Cyankuppler ein solcher einer der Formeln (IIa) und (III) in Kombination mit einem Cyan-DIR-Kuppler der Formel (Ia) verwendet wird und wenn gleichzeitig zugeordnet zu der oder den grünempfindlichen Schichten ein Magentakuppler vom Pyrazoloazoltyp verwendet wird. Solche Magentakuppler sind beispielsweise in US-A 3 725 067 und US-A 4 540 654 beschrieben. Beispiele solcher Kuppler sind Kuppler der allgemeinen Formeln (VI) und (VII)

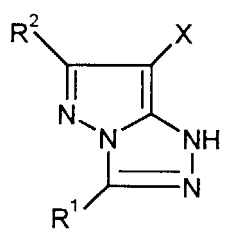

(VI)

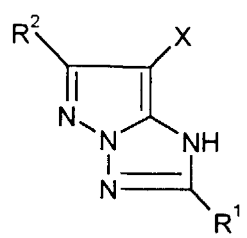

(VII)

worin bedeuten

X H oder eine unter den Bedingungen der Farbentwicklung freisetzbare Gruppe;

$R^1$, $R^2$ H, Alkyl, Aralkyl, Aryl, Alkoxy, Aroxy, Alkylthio, Arylthio, Amino, Anilino, Acylamino, Cyano, Alkoxycarbonyl, Carbamoyl, Sulfamoyl, wobei diese Reste weiter substituiert sein können.

Obwohl die erfindungsgemäß verwendeten Cyan-DIR-Kuppler vorzugsweise einer Silberhalogenidemulsionsschicht mit einer Hauptspektralempfindlichkeit (Rot, Grün, Blau) mit entsprechend zugeordnetem farblosem Farbkuppler, und insbesondere bevorzugt mindestens einer der rotempfindlichen Silberhalogenidemulsionsschichten zugeordnet sind, ist die Erfindung hierauf keineswegs beschränkt. So können die erfindungsgemäß verwendeten Cyan-DIR-Kuppler auch in einer Silberhalogenidemulsionsschicht enthalten sein, die eine von den Hauptspektralempfindlichkeiten (Rot, Grün, Blau) abweichende Spektralempfindlichkeit (Nebenspektralempfindlichkeit) aufweist und die nicht notwendigerweise einen weiteren Farbkuppler enthalten muß. Solche Schichten können beispielsweise durch einen sogenannten Lückensensibilisierungsfarbstoff spektral sensibilisiert sein (EP-A 0 409 019). Durch Zuordnung eines erfindungsgemäß verwendeten Cyan-DIR-Kuppler zu einer solchen Schicht läßt sich die Farbtrennung weiter verbessern (z.B. EP-A 0 167 173). Weiter kann die Hauttonwiedergabe verbessert werden, wenn ein Cyan-DIR-Kuppler der Formel (Ia) einer langrotsensibilisierten Silberhalogenidemulsionsschicht zugeordnet wird.

Die verwendeten Kuppler, d.h. insbesondere die Cyankuppler, beispielsweise der Formeln (IIa) und (III), die Magentakuppler, z.B. 2-Äquivalent- oder 4-Äquivalent-Magentakuppler vom Typ des Pyrazolons oder der Pyrazoloazole, beispielsweise der Formeln (VI) und (VII), wie auch die gegebenenfalls verwendeten farbigen Cyankuppler können in polymerer Form, z.B. als Polymerisatlatex zur Anwendung gelangen.

Hochmolekulare Farbkuppler sind beispielsweise beschrieben in DE-C 1 297 417, DE-A 24 07 569, DE-A 31 48 125, DE-A 32 17 200, DE-A 33 20 079, DE-A 33 24 932, DE-A 33 31 743, DE-A 33 40 376, EP-A 27 284, US-A 4

080 211. Die hochmolekularen Farbkuppler werden in der Regel durch Polymerisation von ethylenisch ungesättigten monomeren Farbkupplern hergestellt.

Die verwendeten Farbkuppler können auch solche sein, die Farbstoffe mit einer schwachen bzw. eingeschränkten Beweglichkeit liefern.

Unter einer schwachen bzw. eingeschränkten Beweglichkeit ist eine Beweglichkeit zu verstehen, die so bemessen ist, daß die Konturen der bei der chromogenen Entwicklung gebildeten diskreten Farbstoffflecken verlaufen und ineinander verschmiert werden. Dieses Ausmaß der Beweglichkeit ist einerseits zu unterscheiden von dem üblichen Fall der völligen Unbeweglichkeit in fotografischen Schichten, der in der herkömmlichen fotografischen Aufzeichnungsmaterialien für die Farbkuppler bzw. die daraus hergestellten Farbstoffe angestrebt wird um eine möglichst hohe Schärfe zu erzielen, und andererseits von dem Fall der völligen Beweglichkeit der Farbstoffe, der beispielsweise bei Farbdiffusionsverfahren angestrebt wird. Die letztgenannten Farbstoffe verfügen meist über mindestens eine Gruppe, die sie im alkalischen Medium löslich machen. Das Ausmaß der erfindungsgemäß angestrebten schwachen Beweglichkeit kann gesteuert werden durch Variation von Substituenten, um beispielsweise die Löslichkeit im organischen Medium des Ölbildners oder die Affinität zur Bindemittelmatrix in gezielter Weise zu beeinflussen.

Über die genannten Bestandteile hinaus kann das farbfotografische Aufzeichnungsmaterial der vorliegenden Erfindung weitere Zusätze enthalten, wie z.B. Antioxidantien, farbstoffstabilisierende Mittel und Mittel zu Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es beispielsweise vorteilhaft, in einer oder mehreren der in dem Aufzeichnungsmaterial enthaltenen Schichten, vorzugsweise in einer der oberen Schichten, UV-absorbierende Verbindungen zu verwenden. Geeignete UV-Absorber sind beispielsweise in US-A 3 253 921, DE-C 2 036 719 und EP-A 0 057 160 beschrieben.

Zur Herstellung farbfotografischer Bilder wird das erfindungsgemäße farbfotografische Aufzeichnungsmaterial, das mindestens eine Silberhalogenidemulsionsschicht und mindestens einen dieser zugeordneten Kuppler der Formel (Ia) enthält, mit einer Farbentwicklerverbindung entwickelt. Als Farbentwicklerverbindung lassen sich sämtliche Entwicklerverbindungen verwenden, die die Fähigkeit besitzen in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl)-3-methylp-phenylendiamin und 1-(N-ethyl-N-methoxyethyl)-3 -methyl-p-phenylendiamin.

Weitere brauchbare Farbentwickler sind beispielsweise beschrieben in J. Amer. Chem. Soc. <u>73,</u> 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind weiterhin Persulfate.

## Beispiel 1

Ein farbfotografisches Aufzeichnungsmaterial für die Colornegativfarbentwicklung wurde herstellt, indem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben. Alle Silberhalogenidemulsionen waren pro 100 g $AgNO_3$ mit 0,1 g 4-Hydroxy-6-methyl-1,3,3a,7-tetraazainden stabilisiert.

Schichtaufbau 1A (Vergleich)

Schicht 1 (rotsensibilisierte Schicht)
rotsensibilisierte Silberbromidiodidemulsion
(4,0 mol-% Iodid; mittlerer Korndurchmesser 0,45 µm) aus

5,35 g $AgNO_3$, mit
3,75 g Gelatine
1,33 g Cyankuppler C-19
1,33 g TKP

0,21 g Dibutylphthalat (DBP)

Schicht 2 (Zwischenschicht)
aus

1,53 g Gelatine
0,74 g Weißkuppler XW-1

Schicht 3 (grünsensibilisierte Schicht)
grünsensibilisierte Silberbromidiodidemulsion
(4,0 mol-% Iodid; mittlerer Korndurchmesser 0,45 µm)
aus

3,10 g $AgNO_3$, mit
2,33 g Gelatine
0,775 g Magentakuppler M-12
0,775 g TKP
0,120 g DBP

Schicht 4 (Zwischenschicht)
aus

1,53 g Gelatine
0,74 g Weißkuppler XW-1

Schicht 5 (Gelbfilterschicht)
gelbes kolloidales Silbersol mit

0,09 g Ag
0,34 g Gelatine

Schicht 6 (blauempfindliche Schicht)
blausensibilisierte Silberbromidiodidemulsion
(4,0 mol-% Iodid; mittlerer Korndurchmesser 0,45 µm)
aus

3,46 g $AgNO_3$, mit
2,98 g Gelatine
1,25 g Gelbkuppler Y-20
1,25 g TKP
0,135 g DBP

Schicht 7 (Schutzschicht)
aus

1,43 g Gelatine

Schicht 8 (Härtungsschicht)
aus

0,68 g Gelatine
0,73 g Härtungsmittel (CAS Reg. No. 65411-60-1)

Die Schichtaufbauten 1B und 1G sind im wesentlichen mit Schichtaufbau 1A identisch, enthielten aber zusätzlich in der 1., 3. und 6. Schicht DIR-Kuppler in den in Tabelle 1 angegebenen, molar äquivalenten Mengen (g).

| Schichtaufbau | 1B | 1C | 1D | 1E | 1F |
|---|---|---|---|---|---|
| DIR-Kuppler | XD-1 | XD-2 | XD-3 | XD-4 | XD-5 |
| | Vergleich | Vergleich | Vergleich | Vergleich | Vergleich |
| 1. Schicht | 0,100 | 0,108 | 0,112 | 0,140 | 0,129 |
| 3. Schicht | 0,058 | 0,063 | 0,065 | 0,081 | 0,074 |
| 6. Schicht | 0,065 | 0,070 | 0,072 | 0,091 | 0,083 |
| Schichtaufbau | 1G | 1H | 1J | | 1K |
| DIR-Kuppler | DIR-1 | DIR-2 | DIR-3 | | DIR-6 |
| | Erfindung | Erfindung | Erfindung | | Erfindung |
| 1. Schicht | 0,113 | 0,119 | 0,120 | | 0,119 |
| 3. Schicht | 0,066 | 0,069 | 0,069 | | 0,069 |
| 6. Schicht | 0,073 | 0,078 | 0,078 | | 0,077 |

In Beispiel 1 wurden folgende Verbindungen verwendet:

XW-1

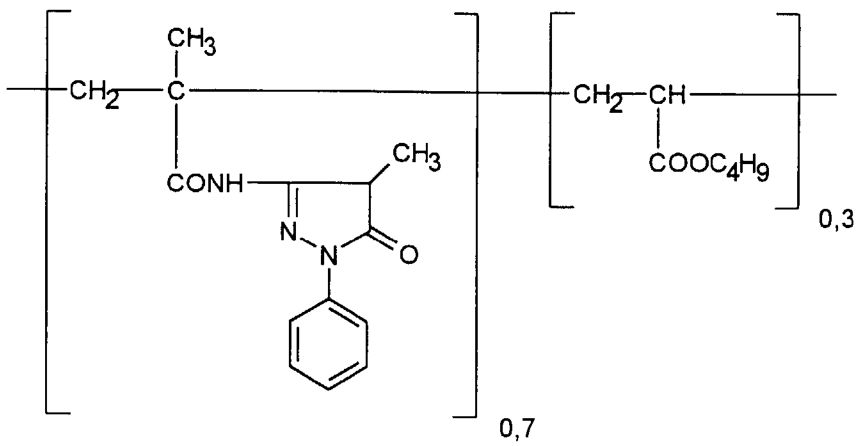

XD-1

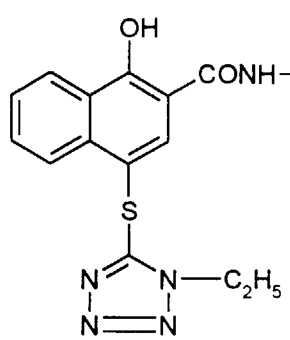

XD-2

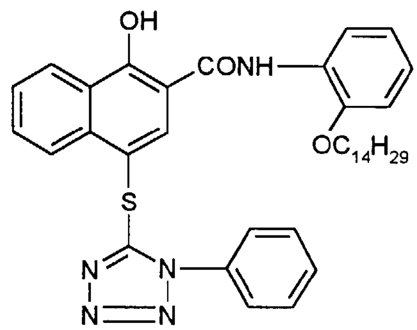

XD-3

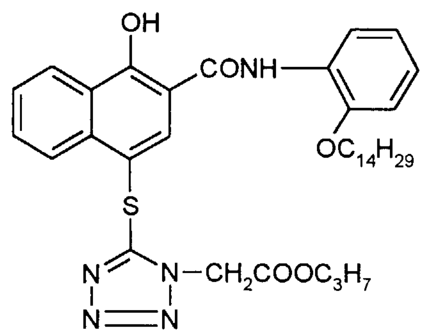

XD-4

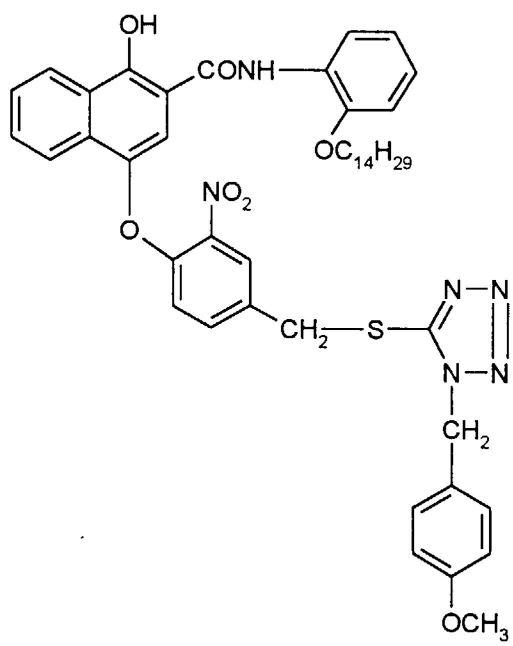

XD-5

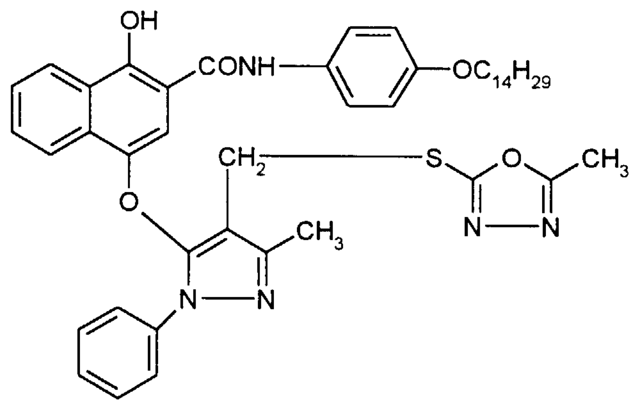

Nach Aufbelichten eines Graukeils mit weißem Licht sowie mit blauem, grünem oder rotem Licht wird die Entwicklung durchgeführt wie beschrieben in "The British Journal of Photography", 1974, Seiten 597 und 598. Die Ergebnisse nach Verarbeitung sind in nachstehender Tabelle wiedergegeben.

| Schichtaufbau | DIR-Kuppler | Inhibierung | | | $\gamma_1$-IIE | | | |
|---|---|---|---|---|---|---|---|---|
| | | gb | pp | bg | gb | pp | bg | |
| 1B | XD-1 | 0,22 | 0,37 | -0,10 | 0,09 | 0,62 | 0,16 | Vergleich |
| 1C | XD-2 | 0,71 | 0,66 | 0,35 | 0,28 | 0,52 | 0,05 | " |
| 1D | XD-3 | 0,32 | 0,51 | 0,08 | 0,09 | 0,76 | 0,12 | " |
| 1E | XD-4 | 0,46 | 0,51 | 0,29 | 0,55 | 0,83 | 0,09 | " |
| 1F | XD-5 | 0,19 | 0,33 | 0,04 | 0,15 | 0,44 | 0,07 | " |
| 1G | DIR-1 | 0,53 | 0,58 | 0,47 | 0,54 | 1,03 | 0,16 | Erfindung |
| 1H | DIR-2 | 0,63 | 0,68 | 0,52 | 0,91 | 1,60 | 0,17 | " |
| 1I | DIR-3 | 0,68 | 0,76 | 0,54 | 1,10 | 1,71 | 0,17 | " |
| 1K | DIR-6 | 0,38 | 0,47 | 0,32 | 0,50 | 0,83 | 0,19 | " |

Die erfindungsgemäßen DIR-Kuppler DIR-1, DIR-2, DIR-3 DIR-6 weisen hohe Werte für die Inhibierung und insbesondere die $\gamma_1$-Interimageeffekte auf.

Die Inhibierung ist ein Maß für den Gradationsrückgang, der durch den DIR-Kuppler hervorgerugen wird. Es ist:

$$\text{Inhibierung} = (\gamma_1 \text{ ohne DIR-Kuppler} - \gamma_1 \text{ mit DIR-Kuppler}) / \gamma_1 \text{ ohne DIR-Kuppler}$$

Der $\gamma_1$-IIE ist ein Maß für die Farbsättigung, die durch DIR-Kuppler erzielt werden kann. Es ist:

$$\gamma_1\text{-IIE} = (\gamma_1 \text{Selektivbelichtung} - \gamma_1 \text{Weißbelichtung} / \gamma_1 \text{Weißbelichtung}$$

Die Vergleichs-DIR-Kuppler XD-1 und XD-2 weisen bg-Kuppler-Strukturen mit herkömmlichen Inhibitoren auf. Die Vergleichs-Verbindung XD-3 zeichnet sich darüberhinaus durch einen im Entwicklerbad desaktivierbaren Inhibitor aus, so daß keine Vergiftung des Entwicklerbades auftreten kann. XD-4 und XD-5 besitzen Timing-Glieder, die durch zeitverzögertes Freisetzen des Inhibitors vergleichsweise hohe Interimage-Effekte bewirken. Eine weitere Möglichkeit, hohe Farbsättigung durch Interimage-Effekte zu erreichen, besteht darin, daß die aus dem DIR-Kuppler freigesetzte Fluchtgruppe den Inhibitor erst durch Reaktion mit Entwickleroxidationsprodukt freigibt, so daß unbelichtete Bereiche des Films ohne Freisetzung des Inhibitors durchquert werden und erst in belichteten Bereichen die Folgereaktion und

damit die Inhibitorwirkung eintritt. Die auf diese Weise erreichte Effizienz muß jedoch mit teuren Synthesekosten und aufgrund des hohen Molgewichtes großen Einsatzmengen erkauft werden. Das erfindungsgemäße Material mit den erfindungsgemäßen Cyan-DIR-Kupplern DIR-1, DIR-2, DIR-3, DIR-6 zeigt bei niedrigen Synthesekosten und geringen Einsatzmengen auch ohne Timing-Glied hervorragende Wirkung auf, wobei der Inhibitor ebenfalls im Entwicklerbad desaktivierbar ist.

**Beispiel 2**

Ein farbfotografisches Aufzeichnungsmaterial für die Colornegativfarbentwicklung wurde hergestellt (Schichtaufbau 2), indem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben. Alle Silberhalogenidemulsionen waren pro 100 g $AgNO_3$ mit 0,5 g 4-Hydroxy-6-methyl-1,3,3a,7-tetraazainden stabilisiert.

Schichtaufbau 2

Schicht 1 (Antihaloschicht)
schwarzes kolloidales Silbersol mit

0,3 gAg
1,2 g Gelatine
0,4 g UV-Absorber UV-1
0,02 g Trikresylphosphat (TKP)

Schicht 2 (Mikrat-Zwischenschicht)
Mikrat-Silberbromidiodidemulsion

(0,5 mol-% Iodid;
mittlerer Korndurchmesser 0,07 µm) aus
0,25 g $AgNO_3$, mit
1,0 g Gelatine

Schicht 3 (1. rotsensibilisierte Schicht, gering empfindlich)
rotsensibilisierte Silberbromidiodidemulsion (4 mol-% Iodid;
mittlerer Korndurchmesser 0,5 µm) aus

2,7 g $AgNO_3$, mit
2,0 g Gelatine
0,88 g Cyankuppler C-9
0,04 g Rotmaske (→cyan) RC-1
0,07 g Gelbmaske (→cyan) YC-1
0,765 g TKP

Schicht 4 (2. rotsensibilisierte Schicht, hochempfindlich)
rotsensibilisierte Silberbromidiodidemulsion
(12 mol-% Iodid;
mittlerer Korndurchmesser 1,0 µm) aus

2,2 g $AgNO_3$, mit
1,8 g Gelatine
0,19 g Cyankuppler C-9
0,17 g TKP

Schicht 5 (Zwischenschicht)

0,4 g Gelatine

0,15 g Weißkuppler XW-1
0,06 g Aluminiumsalz der Aurintricarbonsäure

Schicht 6 (1. grünsensibilisierte Schicht, gering empfindlich) grünsensibilisierte Silberbromidiodidemulsion (4 mol-% Iodid; mittlerer Korndurchmesser 0,35 µm) aus

1,9 g $AgNO_3$, mit
1,8 g Gelatine
0,54 g Magentakuppler M-12
0,065 g Gelbmaske (→magenta) YM-1
0,60 g TKP

Schicht 7 (2. grünsensibilisierte Schicht, hochempfindlich) grünsensibilisierte Silberbromidiodidemulsion (9 mol-% Iodid; mittlerer Korndurchmesser 0,8 µm) aus

1,25 g $AgNO_3$, mit
1,1 g Gelatine
0,195 g Magentakuppler M-12
0,05 g Gelbmaske (→magenta) YM-2
0,60 g TKP

Schicht 8 (Gelbfilterschicht) gelbes kolloidales Silbersol mit

0,09 g Ag
0,25 g Gelatine
0,08 g Scavenger SC-1
0,08 g TKP

Schicht 9 (1. blauempfindliche Schicht, gering empfindlich) blausensibilisierte Silberbromidiodidemulsion (6 mol-% Iodid; mittlerer Korndurchmesser 0,60 µm) aus

0,9 g $AgNO_3$, mit
2,2 g Gelatine
1,1 g Gelbkuppler Y-4
1,1 g TKP

Schicht 10 (2. blauempfindliche Schicht, hochempfindlich) blausensibilisierte Silberbromidiodidemulsion (10 mol-% Iodid; mittlerer Korndurchmesser 1,20 µm) aus

0,6 g $AgNO_3$, mit
0,6 g Gelatine
0,2 g Gelbkuppler Y-4
0,22 g TKP

Schicht 11 (Mikrat-Zwischenschicht) Mikrat-Silberbromidemulsion mittlerer Korndurchmesser 0,06 µm) aus

0,06 g $AgNO_3$, mit
1,0 g Gelatine
0,3 g UV-Absorber UV-2
0,3 g TKP

Schicht (Schutz- und Härtungsschicht) aus

0,25 g Gelatine
0,75 g Härtungsmittel [CAS Reg.Nr. 65411-60-1]

so daß der Gesamtschichtaufbau nach Härtung einen Quellfaktor ≤ 3,5 hatte.

In Beispiel 2 wurden außer den bereits erwähnten Verbindungen folgende Verbindungen verwendet:

UV-Absorber UV-1

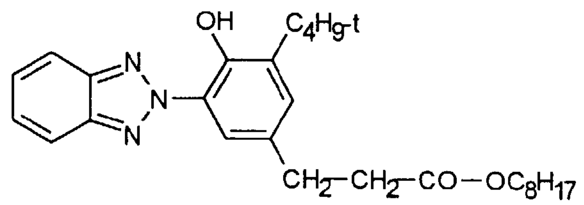

UV-Absorber UV-2

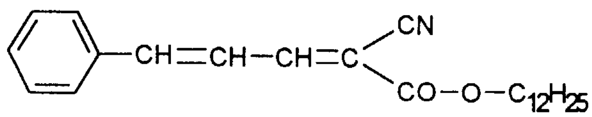

Rotmaske RC-1

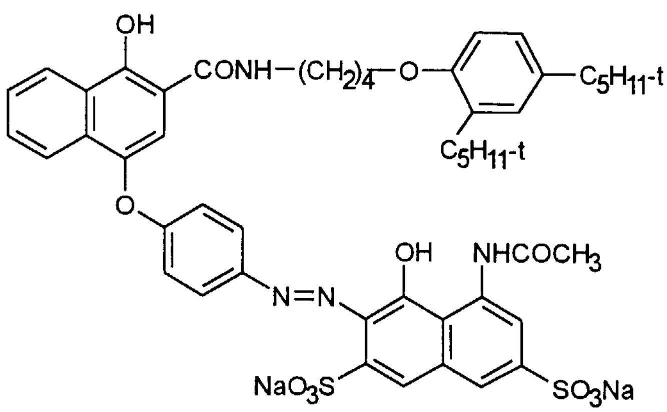

Gelbmaske YC-1

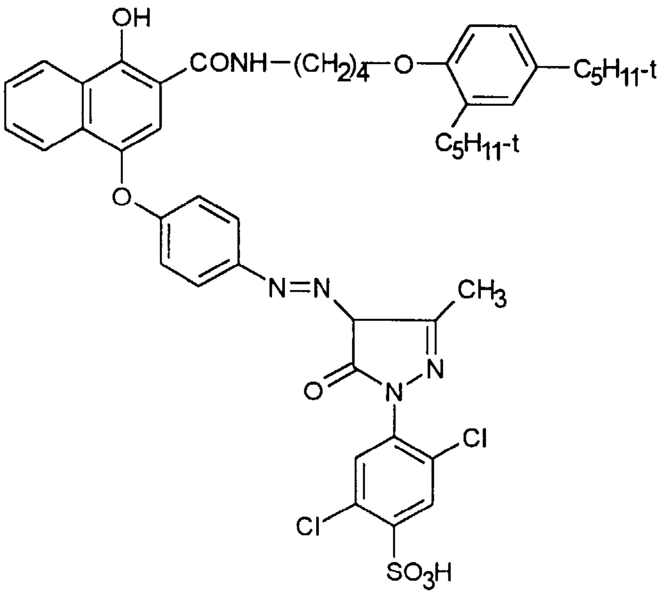

Gelbmaske YM-1

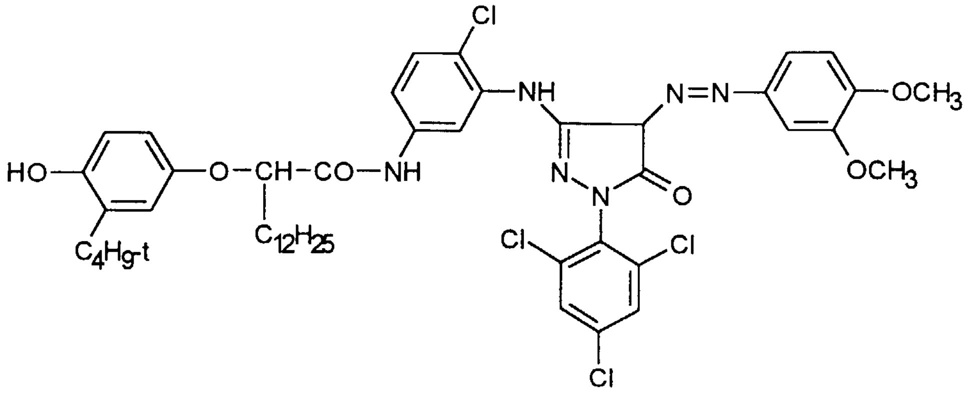

Gelbmaske YM-2

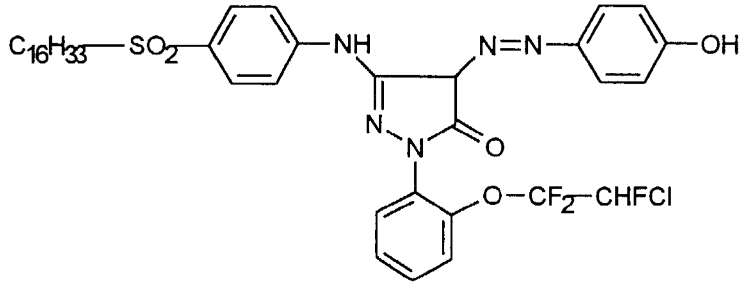

Scavenger SC-1

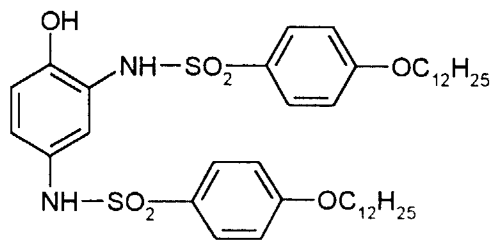

Zusätzlich enthielten die Schichten 3, 6, 9 und 10 DIR-Kuppler gemäß nachstehender Tabelle:

| Schichtaufbau | DIR-Kuppler | in Schicht | | | |
|---|---|---|---|---|---|
| | | 3 | 6 | 9 | 10 |
| 2A (Vergleich | XD-3 | 0,026 g | 0,024 g | 0,037 g | 0,003 g |
| 2B (Vergleich) | XD-5 | 0,027 g | 0,025 g | 0,039 g | 0,003 g |
| 2C (Erfindung) | DIR-1 | 0,027 g | 0,025 g | 0,039 g | 0,003 g |
| 2D (Erfindung) | DIR-2 | 0,025 g | 0,023 g | 0,036 g | 0,003 g |
| 2E (Erfindung) | DIR-3 | 0,029 g | 0,027 g | 0,042 g | 0,003 g |

Die verschiedenen Materialien wurden anschließend hinter einem graduierten Graukeil mit Tageslicht und hinter Blau-, Grün- und Rotfilter belichtet. Danach wurde das Material in einer Durchlauffilmentwicklungsmaschine des Typs CF 35/16 der Agfa Gevaert AG nach dem bei E.CH. Gehret, The British J. ofPhotography 1974, S. 597 beschriebenen Prozeß verarbeitet.

Anschließend wurden folgende $\gamma_1$-IIE-Werte bestimmt:

| **Schichtaufbau** | $\gamma_1$ | | |
|---|---|---|---|
| | **gb** | **pp** | **bg** |
| 2A (Vergleich) | 0,12 | 0,35 | 0,20 |
| 2B (Vergleich) | 0,14 | 0,28 | 0,16 |
| 2C (Erfindung) | 0,33 | 0,38 | 0,35 |
| 2D (Erfindung) | 0,35 | 0,45 | 0,35 |
| 2E (Erfindung) | 0,29 | 0,39 | 0,32 |

Die erfindungsgemäßen Cyan-DIR-Kuppler liefern höhere IIE-Werte als die vergleichbaren bekannten Cyan-DIR-Kuppler.

**Beispiel 3**

Die Schichtaufbauten 3A und 3B (Vergleich) sind identisch mit den Schichtaufbauten 2A und 2C in Beispiel 2. Die Schichtaufbauten 3C (Vergleich) und 3D (Erfindung) weisen im Vergleich zu den Schichtaufbauten 3A und 3B eine zusätzliche Schicht zwischen der 2. und 3. Schicht (im folgenden Schicht 2" genannt) und eine geänderte 3. Schicht auf:

Schichtaufbau 3C

Schicht 2" (Steuerschicht)
Silberbromidiodidemulsion (7 mol-% Iodid, mittlerer Korndurchmesser 0,7 µm, sensibilisiert mit $3{,}7 \cdot 10^{-4}$ mol Sensibilisator S-1 pro 100 g Silbernitrat) aus

1,1g $AgNO_3$, mit
0,83 g Gelatine
0,09 g Cyan-DIR-Kuppler XD-5
0,09 g TKP

Schicht 3 (1. rotsensibilisierte Schicht, gering empfindlich) rotsensibilisierte Silberbromidiodidemulsion (4 mol-% Iodid; mittlerer Korndurchmesser 0,5 µm) aus

2,9 g $AgNO_3$, mit
2,0 g Gelatine
0,95 g Cyankuppler C-9
0,04 g Rotmaske (→cyan) RC-1
0,07 g Gelbmaske (→cyan) YC-1
0,026 g DIR-Kuppler XD-3
0,765 g TKP

Schichtaufbau 3D

Schicht 2" wie in Schichtaufbau 3C, jedoch statt 0,09 g Cyan-DIR-Kuppler XD-5 0,09 g Cyan-DIR-Kuppler DIR-11.

Schicht 3 wie in Schichtaufbau 3C, jedoch statt 0,026 g Cyan-DIR-Kuppler XD-3 0,027 g Cyan-DIR-Kuppler DIR-1.

Mit den so hergestellten Materialien (Schichtaufbauten 3A bis 3D) wurden Testaufnahmen von Personen mit verschiedenen Hauttönen angefertigt. Das Material wurde verarbeitet wie unter Beispiel 2 beschrieben. Kopien wurden auf Colorpapier in der Weise angefertigt, daß eine Vorlage der Graustufe 0,7 auch mit optischer Dichte 0,7 wiedergegeben wurde. Bei der Beurteilung wird eine gelblich braune Hauttonwiedergabe als besser gewertet als eine rötliche Hauttonwiedergabe.

| Schichtaufbau | Steuerschicht 2" | Hauttonwiedergabe |
|---|---|---|
| 3A | ohne | mittel |
| 3B | ohne | mittel |
| 3C | mit | gut |
| 3D | mit | sehr gut |

Wie man sieht, läßt sich besonders mit den erfindungsgemäßen DIR-Kupplern im Zusammenwirken mit einem speziellen Schichtaufbau eine ausgezeichnete Wiedergabe von Hauttönen erreichen.

S-1

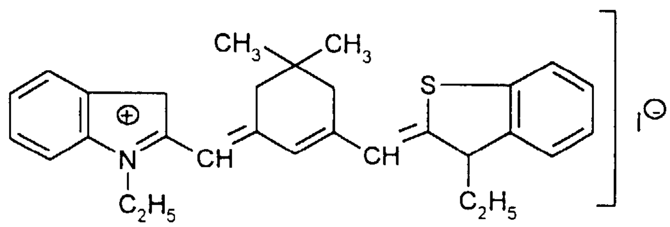

**Patentansprüche**

1. Farbfotografisches Aufzeichnungsmaterial mit mindestens einer auf einen Schichtträger aufgetragenen lichempfindlichen Silberhalogenidemulsionsschicht, der ein farbloser Cyan-DIR-Kuppler zugeordnet ist, dadurch gekennzeichnet, daß der farblose Cyan-DIR-Kuppler der folgenden Formel (Ia) entspricht

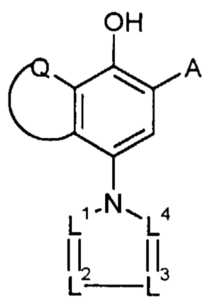

(Ia)

worin bedeuten

| | |
|---|---|
| A | eine Carbamoylgruppe, eine Sulfamoylgruppe oder eine Acylaminogruppe; |
| Q | einen Rest zur Vervollständigung eines ankondensierten Benzolringes; |
| $L^1$, $L^2$, $L^3$, $L^4$ | Ringglieder, von denen zwei für je ein N-Atom und die beiden anderen für je eine Gruppe |

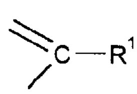

stehen, worin $R^1$ für H, Alkyl, Alkylthio, Aryl, eine heterocyclische Gruppe oder -COOR$^2$ (mit $R^2$ = Alkyl oder Aryl) steht.

2. Aufzeichnungsmaterial nach Anspruch 1, dadurch gekennzeichnet, daß der aus dem farblosen Cyan-DIR-Kuppler der Formel Ia freigesetzte Inhibitor mindestens eine der folgenden Besonderheiten aufweist:

   a) Er enthält eine im alkalischen Entwickler verseifbare Gruppe.

   b) Er hat eine diffusibility ≥ 0,4.

3. Aufzeichnungsmaterial nach einem der Ansprüche 1 und 2, dadurch gekennzeichnet, daß es mindestens eine rotempfindliche Silberhalogenidemulsionsschicht mit einem farblosen Cyankuppler, mindestens eine grünempfindliche Silberhalogenidemulsionsschicht mit einem farblosen Magentakuppler und mindestens eine blauempfindliche Silberhalogenidemulsionsschicht mit einem farblosen Gelbkuppler enthält.

4. Aufzeichnungsmaterial nach Anspruch 3, dadurch gekennzeichnet, daß der farblose Cyankuppler einer der Formeln IIa und III entspricht

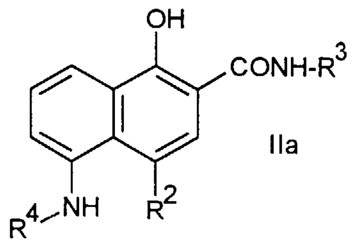

IIa

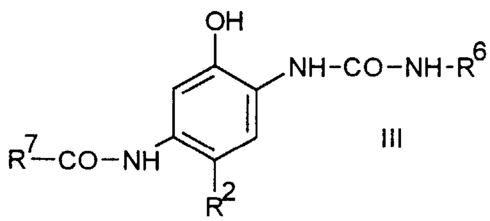

III

worin bedeuten

$R^2$ H oder eine unter den Bedingungen der Farbentwicklung freisetzbare Gruppe, die dem Kuppler keine Farbe verleiht;

$R^3$ Alkyl oder Aryl;

$R^4$ H, Acyl, wobei der Acylrest sich von aliphatischen oder aromatischen Carbon- oder Sulfonsäuren, von N-substituierten Carbamin- oder Sulfaminsäuren oder von Kohlensäurehalbestern ableitet,
oder

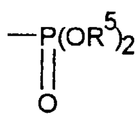

oder

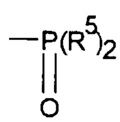

$R^5$ Alkyl;

$R^6$ eine heterocyclische Gruppe oder Aryl;

$R^7$ einen Ballastrest.

5. Aufzeichnungsmaterial nach einem der Ansprüche 3 und 4, dadurch gekennzeichnet, daß der farblose Cyan-DIR-Kuppler zusammen mit dem farblosen Cyankuppler in einer rotempfindlichen Silberhalogenidemulsionsschicht enthalten ist.

6. Aufzeichnungsmaterial nach einem der Ansprüche 4 und 5, dadurch gekennzeichnet, daß dem farblosen Cyankuppler ein roter Cyankuppler und/oder ein gelber Cyankuppler zugeordnet sind/ist.

7. Aufzeichnungsmaterial nach Anspruch 5, dadurch gekennzeichnet, daß mindestens eine blauempfindliche Silberhalogenidemulsionsschicht und/oder mindestens eine grünempfindliche Silberhalogenidemulsionsschicht einen farblosen Cyan-DIR-Kuppler der Formel Ia enthält.

**Claims**

1. Colour photographic recording material having at least one photosensitive silver halide emulsion layer applied onto a layer support, which layer is associated with a colourless cyan DIR coupler, characterised in that the colourless cyan DIR coupler is of the following formula (Ia)

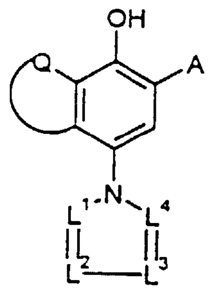

(Ia)

in which

A means a carbamoyl group, a sulfamoyl group or an acylamino group;

Q means a residue to complete a fused benzene ring;

$L^1$, $L^2$, $L^3$, $L^4$ mean ring members, two of which each denote an N atom and the other two each denote a group

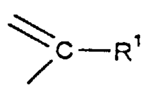 ,

in which $R^1$ denotes H, alkyl, alkylthio, aryl, a heterocyclic group or -$COOR^2$ (where $R^2$ = alkyl or aryl).

**2.** Recording material according to claim 1, characterised in that the inhibitor released from the colourless cyan DIR coupler of the formula Ia exhibits at least one of the following particular features:

a) it contains a group which is saponifiable in the alkaline developer.

b) it has a diffusibility of ≥ 0.4.

**3.** Recording material according to one of claims 1 and 2, characterised in that it contains at least one red-sensitive silver halide emulsion layer containing a colourless cyan coupler, at least one green-sensitive silver halide emulsion layer containing a colourless magenta coupler and at least one blue-sensitive silver halide emulsion layer containing a colourless yellow coupler.

**4.** Recording material according to claim 3, characterised in that the colourless cyan coupler is of one of the formulae IIa and III

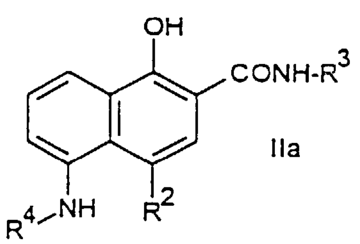

IIa

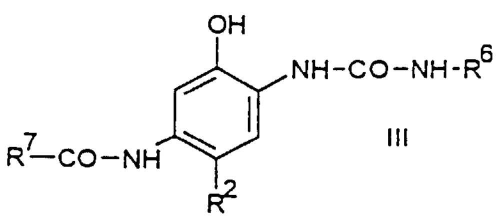

III

in which

$R^2$ means H or a group which is releasable under colour development conditions and imparts no colour to the coupler;

$R^3$ means alkyl or aryl;

$R^4$ means H, acyl, wherein the acyl residue is derived from aliphatic or aromatic carboxylic or sulfonic acids, from N-substituted carbamic or sulfamic acids or from carbonic acid semi-esters, or

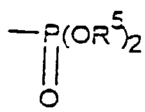 or 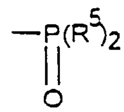

$R^5$ means alkyl;

$R^6$ means a heterocyclic group or aryl;

$R^7$ means a ballast residue.

**5.** Recording material according to one of claims 3 and 4, characterised in that a red-sensitive silver halide emulsion layer contains the colourless cyan DIR coupler together with the colourless cyan coupler.

**6.** Recording material according to one of claims 4 and 5, characterised in that a red cyan coupler and/or a yellow cyan coupler is/are associated with the colourless cyan coupler.

**7.** Recording material according to claim 5, characterised in that at least one blue-sensitive silver halide emulsion layer and/or at least one green-sensitive silver halide emulsion layer contains a colourless cyan DIR coupler of the formula Ia.

## Revendications

**1.** Matériau d'enregistrement photographique couleur ayant au moins une couche d'émulsion d'halogénure d'argent sensible à la lumière appliquée sur un support de couches, à laquelle est associé un coupleur DIR cyan incolore, caractérisé en ce que le coupleur DIR cyan incolore correspond à la formule (Ia) suivante

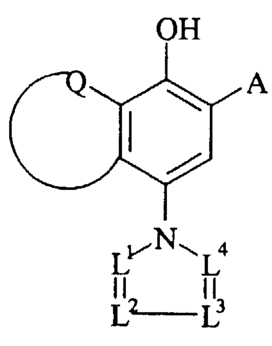

(Ia)

où

| | |
|---|---|
| A | représente un groupe carbamoyle, un groupe sulfamoyle ou un groupe acylamino ; |
| Q | représente un reste pour former un cycle benzénique condensé ; |
| $L^1$, $L^2$, $L^3$, $L^4$ | représentent des éléments cycliques parmi lesquels deux représentent chacun un atome d'azote et les deux autres représentent chacun un groupe |

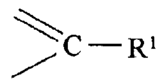

où

$R^1$ représente H, alkyle, alkylthio, aryle, un groupe hétérocyclique ou $-COOR^2$ (avec $R^2$ = alkyle ou aryle).

**2.** Matériau d'enregistrement selon la revendication 1, caractérisé en ce que l'inhibiteur libéré par le coupleur DIR cyan incolore de formule (Ia) présente au moins l'une des particularités suivantes :

a) il contient un groupe saponifiable dans le révélateur alcalin,
b) il a une diffusibilité ≥ 0,4.

**3.** Matériau d'enregistrement selon l'une des revendications 1 et 2, caractérisé en ce qu'il contient au moins une couche d'émulsion d'halogénure d'argent sensible au rouge avec coupleur cyan incolore, au moins une couche d'émulsion d'halogénure d'argent sensible au vert avec un coupleur magenta incolore et au moins une couche d'émulsion d'halogénure d'argent sensible au bleu avec un coupleur jaune incolore.

**4.** Matériau d'enregistrement selon la revendication 3, caractérisé en ce que le coupleur cyan incolore correspond à l'une des formules (IIa) et (III)

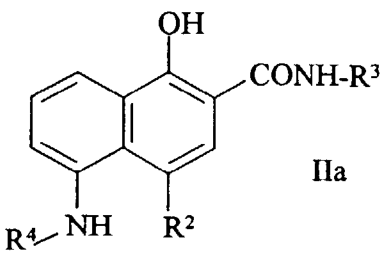

IIa

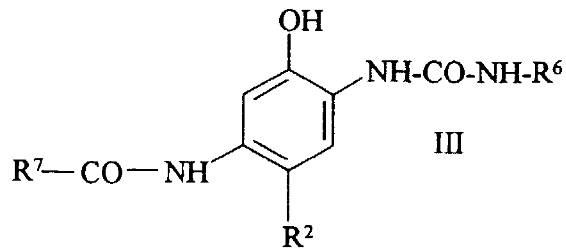

III

où

$R^2$ représente H ou un groupe libérable dans les conditions du développement chromogène, qui ne communique aucune couleur au coupleur ;
$R^3$ représente alkyle ou aryle ;
$R^4$ représente H, acyle, le reste acyle étant issu d'acides carboxyliques ou sulfoniques aliphatiques ou aromatiques, d'acides carbamiques ou sulfamiques N-substitués ou d'hémiesters de l'acide carbonique, ou bien

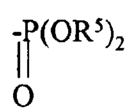 ou 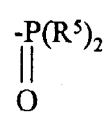 ;

$R^5$ représente alkyle ;
$R^6$ représente un groupe hétérocyclique ou aryle ;
$R^7$ représente un reste ballast.

**5.** Matériau d'enregistrement selon l'une des revendications 3 et 4, caractérisé en ce que le coupleur DIR cyan incolore est contenu en même temps que le coupleur cyan incolore dans une couche d'émulsion d'halogénure d'argent sensible au rouge.

6. Matériau d'enregistrement selon l'une des revendications 4 et 5, caractérisé en ce qu'un coupleur cyan rouge et/ou un coupleur cyan jaune est/sont associés au coupleur cyan incolore.

7. Matériau d'enregistrement selon la revendication 5, caractérisé en ce qu'au moins une couche d'émulsion d'halogénure d'argent sensible au bleu et/ou au moins une couche d'émulsion d'halogénure d'argent sensible au vert contiennent un coupleur DIR cyan incolore de formule (Ia).